# EUROPÄISCHE PATENTSCHRIFT

(11) **EP 3 506 878 B1**
(45) Veröffentlichungstag und Bekanntmachung des Hinweises auf die Patenterteilung: **05.08.2020**
(21) Anmeldenummer: 17734306.8
(22) Anmeldetag: 30.06.2017
(51) Int. Cl.: A61K 8/44, A61K 8/64, A61K 8/46, A61Q 5/04

(54) **PERMANENTE HAARVERFORMUNGSMITTEL MIT BUNTE-SALZEN VON AMINOSÄUREN UND OLIGOPEPTIDEN**
PERMANENT HAIR WAVING AGENTS WITH "BUNTE-SALTS" OF AMINO ACIDS AND OLIGOPEPTIDES
COMPOSITION POUR LA DÉFORMATION PERMANENTE DES FIBRES KÉRATINIQUES AVEC "BUNTE"-SELS" D'ACIDES AMINÉS ET OLIGOPEPTIDES

(30) Priorität: 05.09.2016 DE 102016216761
(43) Veröffentlichungstag der Anmeldung: 10.07.2019
(73) Patentinhaber: Henkel AG & Co. KGaA, 40589 Düsseldorf (DE)
(72) Erfinder: KNÜBEL, Georg, 40219 Düsseldorf (DE); FÖRSTER, Thomas, 40591 Düsseldorf (DE); FLOUW, Romain, 158 0094 Tokyo-to Setagaya-ku (JP)
(86) Internationale Anmeldenummer: PCT/EP2017/066250
(87) Internationale Veröffentlichungsnummer: WO 2018/041439

(56) Entgegenhaltungen:
- WO-A1-00/32156
- JP-A- 2006 265 201

## Beschreibung

Die vorliegende Erfindung liegt auf dem Gebiet der Kosmetik und betrifft eine Mehrkomponenten-Verpackungs-Einheit (Kit-of-Parts) zur permanenten Formveränderung von keratinischen Fasern, welche mindestens drei Container mit mindestens drei getrennt voneinander konfektionierten Zubereitungen umfasst. Bei der ersten Zubereitung handelt es sich ein erstes kosmetisches Mittel (A), welches mindestens eine Keratin reduzierende Verbindung enthält. Die zweite Zubereitung stellt ein zweites kosmetisches Mittel (B) dar, welches mindestens ein Bunte-Salze einer Aminosäure bzw. eines Oligopepdits einer Formel (I) enthält.

Ein weiterer Gegenstand der vorliegenden Erfindung ist ein Verfahren zur dauerhaften Haarverformung, bei welchem die zuvor beschriebene Mehrkomponenten-Verpackungseinheit zum Einsatz kommt. Ein weiterer Gegenstand der vorliegenden Erfindung ist die Verwendung der Bunte-Salze der Formel (I) zur Verminderung der durch Keratin reduzierende Verbindungen verursachten Haarschädigung.

Eine dauerhafte Formveränderung von keratinischen Fasern wird üblicherweise so durchgeführt, dass die Faser mechanisch verformt und die Verformung durch geeignete Hilfsmittel (beispielsweise Wickler oder Papilloten) festgelegt wird. Vor und/oder nach dieser Verformung behandelt man die Faser mit der wässrigen Zubereitung einer Keratin reduzierenden Substanz und spült nach einer Einwirkungszeit mit Wasser oder einer wässrigen Lösung. Im Anschluss daran behandelt man dann die Faser mit der wässrigen Zubereitung eines Oxidationsmittels. Nach einer Einwirkungszeit wird auch dieses ausgespült und die Faser von den mechanischen Verformungshilfsmitteln befreit.

Eine gegenüber Wassereinfluss stabile, permanente Formveränderung der Keratinfasern kann nur durch chemische Eingriffe in das Haar erreicht werden, die auf eine Spaltung der im Haarkeratin befindlichen Cystin-Disulfid-Bindungen abzielen. Diese Spaltung erfolgt meist unter Einwirkung von Keratin reduzierenden Substanzen. Hierbei handelt es sich um Reduktionsmittel, die einen Teil der Disulfid-Bindungen des Keratins zu SH-Gruppen spalten, so dass es zu einer Lockerung der Peptidvernetzung kommt. Bedingt durch die Spannung der auf einen Wickler aufgewickelten Keratinfasern kommt es zu einer Neuorientierung des Keratingefüges. Unter dem Einfluss des Oxidationsmittels werden erneut Disulfid-Bindungen geknüpft, und auf diese Weise wird das Keratingefüge in der vorgegebenen Verformung neu fixiert. Ein bekanntes derartiges Verfahren stellt die Dauerwell-Behandlung menschlicher Haare dar. Dieses kann zur Erzeugung von Locken und Wellen in glattem Haar angewendet werden.

In analoger Weise können die zuvor beschriebenen Mittel auch zur Glättung von gekräuselten Haaren eingesetzt werden. In diesem Fall erfolgt die mechanische Verformung jedoch mittels Einsatz eines Kammes oder einer Bürste.

Nach der Dauerwellbehandlung resultiert ein gelocktes (bzw. geglättetes) Haar, das innerlich unter Spannung steht, neue (reduktiv bzw. oxidativ veränderte) Aminosäuren enthält und unvollständig quervernetzt ist. Dies kann zur allmählichen, langsamen Relaxation der Verformung führen, d.h. die Locke hängt sich aus. Eine weitere negative Begleiterscheinung der dauergewellten Haare ist oftmals eine Schädigung der Haarstruktur, da sowohl das Haarinnere als auch die Oberfläche des Haares durch die Kombination aus Reduktions- und Oxidationsschritt hydrophiler werden. Aus diesem Grund können hohe Luftfeuchte, häufiges Haarewaschen oder auch Kämmen und Bürsten ein Nachlassen der Wellwirkung auf den geschädigten Haaren führen. Nach wie vor wird nach neuen Mitteln bzw. Dauerwellverfahren gesucht, mit denen sich all diese bekannten Nachteile vermeiden lassen.

Es hat in der Vergangenheit nicht an Versuchen gefehlt, Wellmittel zu formulieren, welche die Struktur der Haarfaser schonen, mild zur Kopfhaut sind und ein hervorragendes Wellergebnis liefern. Die bislang bekannten Mittel können jedoch alle noch nicht als optimal bezeichnet werden.

Die Aufgabe der vorliegenden Erfindung bestand im Auffinden von neuen Mitteln bzw. neuen Verfahren zur dauerhaften Verformung von keratinischen Fasern, welche eine verstärkte, lang anhaltende Wellwirkung besitzen und hierbei gleichzeitig eine maximal möglichen Faserschutz auf den Haaren entfalten.

Überraschenderweise hat sich nun herausgestellt, dass diese Aufgabe optimal gelöst werden kann, wenn die Keratinfasern unter Einsatz von Produkten permanent verformt werden, die neben den bekannten Keratin reduzierenden Verbindungen, die insbesondere aus der Gruppe der Thiole und/oder Disulfide ausgewählt werden, zusätzlich mindestens ein Bunte-Salz einer Aminosäure, eines Oligopeptids und/oder eines Peptids umfassen. Hierbei werden die Keratin reduzierende Verbindung und das Bunte-Salze getrennt konfektioniert in Form von mindestens zwei kosmetischen Mitteln zur Verfügung gestellt, die dem Anwender in Form einer Mehrkomponenten-Verpackungseinheit angeboten werden.

Ein erster Gegenstand der Erfindung ist eine Mehrkomponenten-Verpackungseinheit (Kit-of-Parts), wie in den Ansprüchen definiert, zur permanenten Formveränderung von keratinischen Fasern, insbesondere menschlichen Haaren, umfassend getrennt voneinander konfektioniert
- einen Container (i) enthaltend ein kosmetisches Mittel (A) und
- einen Container (ii) enthaltend ein kosmetisches Mittel (B), und
- einen Container (iii) enthaltend ein kosmetisches Mittel (C),
wobei das Mittel (A) in Container (i) Wasser enthält, einen pH-Wert von 7. 5 bis 9. 5 besitzt und mindestens eine Keratin reduzierende Verbindungen enthält, und
das Mittel (B) in Container (ii) Wasser enthält, einen pH-Wert von 3. 0 bis 5. 5 besitzt und mindestens eine Verbindung der allgemeinen Formel (I) enthält 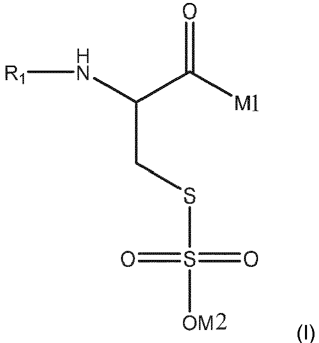 wobei
R1 steht für ein Wasserstoffatom oder für ein Strukturelement der Formel (II) 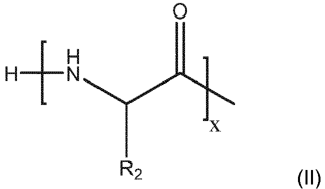 wobei
x für eine ganze Zahl von 1 bis 100 steht,
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 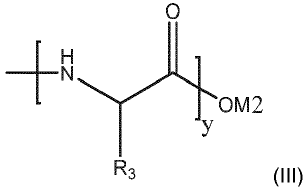
wobei
y für eine ganze Zahl von 1 bis 100 steht
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺, und- das Mittel (C) in Container (iii) Wasser enthält, einen pH-Wert von 2. 5 bis 4,5 besitzt und mindestens ein Oxidationsmittel enthält.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zur permanenten Formveränderung von Keratinfasern geeignet sind, steht prinzipiell einer Verwendung auch auf anderen Gebieten nichts entgegen.

Unter der permanenten Formveränderung von keratinischen Fasern wird eine Formveränderung verstanden, bei dem zunächst ein Reduktionsmittel und dann ein Oxidationsmittel auf den Keratinfasern angewendet werden. Durch den Einfluss des Reduktionsmittels - das auch als Keratin reduzierende Verbindung bezeichnet wird - erfolgt zunächst die reduktive Spaltung der im Haar befindlichen Cystin-Disulfid-Bindungen. Während des Verformungsprozesses wird das Haar unter Zuhilfenahme mechanischer Verformungshilfsmittel (z.B. Lockenwickler, Papilloten usw.) in deine neue Form gebracht. Die mechanische Verformung des Haares kann hierbei vor, während oder nach dem Aufbringen des Reduktionsmittels erfolgen.

Durch späteres Auftragen des Oxidationsmittels werden in den verformten Keratinfasern neue Disulfid-Bindungen gebildet und die Keratinfasern auf diese Weise fixiert. Die permanente Formveränderung übersteht in aller Regel mehrere Haarwäschen.

Ein Mittel zur permanenten Formveränderung im Sinne der vorliegenden Erfindung kann sowohl zur Erzeugung von Locken (in diesem Fall werden als mechanische Verformungshilfsmittel beispielsweise Lockenwickler eingesetzt) oder aber auch zur Glättung (hier erfolgt die mechanische Verformung z.B. mittels eine Kamms) angewendet werden.

Die erfindungsgemäße Mehrkomponenten-Verpackungseinheit umfasst mindestens zwei getrennt konfektionierte Container (i) und (ii). Bei diesen Containern kann es sich beispielsweise um Tuben Flaschen, Sachets, Beutel, Dosen oder auch jedes andere Behältnis handeln, das zur Aufbewahrung eines kosmetischen Mittels geeignet ist. Die Container können beispielsweise aus Kunststoff, Metall, Glas oder auch aus Verbundwerkstoffen aus den vorgenannten Materialien gefertigt sein. Der erste Container (i) enthält ein erstes kosmetisches Mittel (A). Der zweite Container (ii) enthält ein zweites kosmetisches Mittel (B).

Weiterhin kann die erfindungsgemäße Mehrkomponenten-Verpackungseinheit auch noch weitere Container, z.B. einen dritten Container (iii), oder einen dritten und vierten Container (iii) und (iv) umfassen. Der optional enthaltene dritte Container (iii) enthält ein drittes Mittel (C). Der optional enthaltene vierte Container (iv) enthält ein viertes kosmetisches Mittel (D).

Das Mittel (A) und das Mittel (B) (sowie ggf. die Mittel (C) und (D)) enthalten die erfindungswesentlichen Verbindungen jeweils in einem kosmetischen Träger, bevorzugt in einem geeigneten wässrigen, alkoholischen oder wässrig-alkoholischen Träger. Zum Zwecke der Haarbehandlung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel. Der wässriger Träger enthält im Sinne der Erfindung mindestens 30 Gew.-%, insbesondere mindestens 50 Gew.-% Wasser, bezogen auf das Gesamtgewicht der Mittel. Erfindungsgemäß bevorzugt sind wässrige Träger.

### Keratin reduzierende Verbindungen in Mittel (A)

Das Mittel (A) ist erfindungsgemäß dadurch gekennzeichnet, dass es mindestens eine Keratin reduzierende Verbindung enthält. Unter Keratin reduzierenden Verbindungen werden die Reduktionsmittel verstanden, deren Reduktionspotential ausreichend hoch ist, um die in den Keratinfasern enthaltenen Cystin-Disulfid-Bindungen reduktiv zu spalten und in ihre entsprechenden Thiole zu überführen. Die Keratin reduzierende Verbindungen im Sinne der vorliegenden Erfindung sind kosmetisch verträglich.

Bei den Keratin reduzierenden Verbindungen handelt es sich insbesondere um organische Verbindungen, die mindestens eine Thiolgruppe (-SH) und/oder mindestens eine Disulfidgruppierung (-S-S-) besitzen.

Die Keratin reduzierenden Verbindungen sind somit organische Verbindungen, die dadurch gekennzeichnet sind, dass sie als Strukturbestandteile eine Thiolgruppe bzw. Mercaptogruppe (-SH) und/oder eine Disulfidgruppe (-S-S-) besitzen.

Neben der Thiolgruppe und/oder der Disulfidgruppe können die keratinreduzierenden Verbindungen auch noch weitere Substituenten besitzen, bei denen es sich um jeweils eine oder mehrere Aminogruppen, Hydroxygruppen, Säuregruppen, Amidgruppen oder Estergruppen handeln kann. Die im Mittel (A) enthaltenen Keratin reduzierenden Verbindungen fallen erfindungsgemäß nicht unter die Gruppe der Bunte-Salze (a) der Formel (I), sondern sind von diesen verschieden.

Ein erster Gegenstand der Erfindung ist mit anderen Worten eine Mehrkomponenten-Verpackungseinheit (Kit-of-Parts) zur permanenten Formveränderung von keratinischen Fasern, insbesondere menschlichen Haaren, umfassend getrennt voneinander konfektioniert
- einen Container (i) enthaltend ein kosmetisches Mittel (A) und
- einen Container (ii) enthaltend ein kosmetisches Mittel (B),
   wobei
- das Mittel (A) in Container (i) mindestens eine organische Keratin reduzierende Verbindung mit mindestens einer Thiolgruppe (-SH) und/oder mindestens einer Disulfidgruppe (-S-S-) enthält und
- das Mittel (B) in Container (ii) mindestens eine Verbindung der allgemeinen Formel (I) enthält, 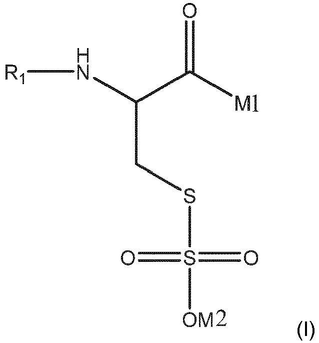 wobei
   R1 steht für ein Wasserstoffatom oder für ein Strukturelement der Formel (II) 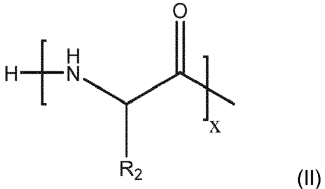 wobei
   x für eine ganze Zahl von 1 bis 100 steht,
   der Rest R2 in jedem der Strukturelemente der Formel (II) jeweils unabhängig vom vorangegangenen Strukturelement der Formel (II) gewählt werden kann,
   R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   M1 steht für die Gruppierung -OM2 oder für ein Strukturelement der Formel (III) 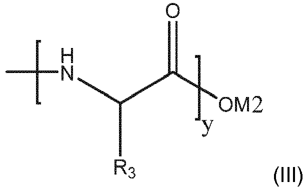
   wobei
   y für eine ganze Zahl von 1 bis 100 steht
   der Rest R3 in jedem der Strukturelemente der Formel (III) jeweils unabhängig vom vorangegangenen Strukturelement der Formel (III) gewählt werden kann,
   R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   M2 steht für ein Wasserstoffatom, ein Äquivalent eines ein oder mehrwertigen Kations oder ein Ammoniumion (NH₄)⁺.

Geeignete Keratin reduzierende Verbindungen sind beispielsweise Thioglycolsäure, Thiomilchsäure, Thioäpfelsäure, Cystein, Cysteamin, Cystin, 2-Mercaptoethansulfonsäure und/oder auch die phyiologisch verträglichen Salze der vorgenannten Verbindungen. Unter einem physiologisch verträglichen Salz wird ein Salz der vorgenannten Verbindungen verstanden, die jeweils in ihre ionische Form überführt wurden und die ein physiologisch verträgliches Gegenion besitzen. Ein physiologisch verträgliches Salz kann auf die Haare bzw. auf die Haut appliziert werden, ohne dass es zu toxikologischen Nachteilen kommt, d.h. ein physiologisch verträgliches Salz eines vorgenannten Reduktionsmittels hat physiologisch keine negativeren Auswirkungen als das Reduktionsmittel selbst. Physiologisch verträgliche Salze sind beispielsweise die Alkalimetallsalze, die Erdalkalimetallsalze oder die Ammoniumsalze der vorgenannten Säuren. Ein physiologisch verträgliches Salz von Cysteamin ist beispielsweise sein Hydrochlorid oder Hydrobromid.

In einer weiteren ganz besonders bevorzugten Ausführungsform ist eine erfindungsgemäße Mehrkomponenten-Verpackungseinheit dadurch gekennzeichnet, dass das Mittel (A) in Container (i) mindestens eine Keratin reduzierende Verbindung aus der Gruppe aus Thioglycolsäure, Thiomilchsäure, Thioäpfelsäure, Cystein, Cysteamin, Cystin, 2-Mercaptoethansulfonsäure und/oder deren physiologisch verträglichen Salzen enthält.

Thioglycolsäure wird alternativ auch als Sulfanylessigsäure oder Mercaptoessigsäure bezeichnet und besitzt die CAS-Nummer 68-11-1. Geeignete physiologisch verträgliche Salze sind beispielsweise das Natriumsalz, das Kaliumsalz oder das Ammoniumsalz der Thioglycolsäure.

Bei Ammoniumthioglycolat handelt es sich um das Ammoniumsalz der Thiglycolsäure (d.h. das Ammoniumsalz der Sulfanylessigsäure) (Formel i)

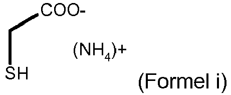

Unter Thiomilchsäure wird D-Thio-Milchsäure, L-Thio-Milchsäure und/oder deren Gemisch verstanden. Thiomilchsäure wird alternativ auch als 2-Mercaptopropionsäure bezeichnet und besitzt die CAS-Nummer 79-42-5. Geeignete physiologisch verträgliche Salze sind beispielsweise das Natriumsalz, das Kaliumsalz oder das Ammoniumsalz der Thiomilchsäure.

Bei Ammoniumthiolactat handelt es sich um das Ammoniumsalz der Thiomilchsäure (d.h. das Ammoniumsalze der 2-Sulfanylpropionsäure) (Formel ii).

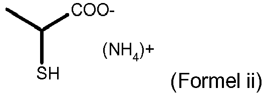

Bei Thioapfelsäure handelt es sich um eine Verbindung der Formel (iii). Unter Thioapfelsäure wird D-Thio-Äpfelsäure, L-Thio-Äpfelsäure und/oder deren Gemisch verstanden. Geeignete physiologisch verträgliche Salze sind das Natriumsalz, das Kaliumsalz oder das Ammoniumsalz von Thioapfelsäure.

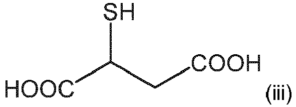

Unter Cystein wird D-Cystein, L-Cystein und/oder deren Gemisch verstanden. Cystein wird alternativ auch als 2-Amino-3-mercapto-propansäure oder 2-Amino-3-sulfanylpropansäure bezeichnet. Geeignete physiologisch verträgliche Salze sind das Natriumsalz, das Kaliumsalz oder das Ammoniumsalz von Cystein.

Cysteamin wird alternativ auch als 2-Aminoethanol bezeichnet und besitzt die Formel HS-CH₂-CH₂-NH₂. Geeignete physiologisch verträgliche Salze von Cysteamin sind beispielsweise das Hydrochlorid, das Hydrobromid, das Sulfat oder das Hemisulfat.

Cystin ist ein Disulfid, das durch Oxidation von zwei Molekülen der Aminosäure Cystein entsteht. Cystin besitzt die CAS-Nummer 56-89-3. Von der Definition des Cystins mit umfasst sind die (D,D)- , (D,L)-, (L,D)- und die (L,L)-Isomere. Geeignete physiologisch verträgliche Salze sind das Natriumsalz, das Kaliumsalz oder das Ammoniumsalz von Cystin.

2-Mercaptoethansulfonsäure ist eine Verbindung der Formel (IV). Geeignete physiologisch verträgliche Salze sind beispielsweise das Natriumsalz, das Kaliumsalz oder das Ammoniumsalz der 2-Mercaptoethansulfonsäure.

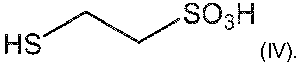

Die vorgenannten Reduktionsmittel sind kommerziell bei verschiedenen Anbietern (Sigma Aldrich, Merck, Fluka oder Alfa Aesar usw.) erhältlich.

Es hat sich herausgestellt, dass bestimmte Keratin reduzierende Verbindungen besonders gut mit den Bunte-Salzen der Formel (I) kompatibel sind. Ganz besonders bevorzugt sind Thioglycolsäure, Ammoniumthioglycolat, Thiomilchsäure und/oder Ammoniumthiolactat, da diese Reduktionsmittel, wenn sie vor, nach oder auch zusammen mit den Bunte Salzen der Formel (I) innerhalb eines permanenten Formveränderungsprozesses angewendet werden, zu einer besonders starken Verbesserung der Wellwirkung bei bestmöglichem Faserschutz führten.

In einer weiteren ganz besonders bevorzugten Ausführungsform ist eine erfindungsgemäße Mehrkomponenten-Verpackungseinheit dadurch gekennzeichnet, dass das Mittel (A) in Container (i) eine oder mehrere Keratin reduzierende Verbindungen aus der Gruppe aus Thioglycolsäure, Ammoniumthioglycolat, Thiomilchsäure und/oder Ammoniumthiolactat enthält.

Das Mittel (A) enthält eine oder mehrere Keratin reduzierende Verbindungen bevorzugt in bestimmten Mengenbereichen, um eine bestmögliche Verformung bei geringstmöglicher Schädigung zu gewährleisten. Besonders bevorzugt enthält das Mittel (A) eine oder mehrere Keratin reduzierende Verbindungen in einer Gesamtmenge von 1,5 bis 20,0 Gew.-%, bevorzugt 3,0 bis 15,0 Gew.-%, weiter bevorzugt von 4,0 bis 12,0 Gew.-% und besonders bevorzugt von 4,5 bis 12,5 Gew.-%. Hierbei sind alle Mengenangaben in Gew.-% auf das Gesamtgewicht der im Mittel enthaltenen Keratin reduzierenden Verbindungen bezogen, das zum Gesamtgewicht das Mittels (A) in Relation gesetzt wird.

In einer weiteren ganz besonders bevorzugten Ausführungsform ist eine erfindungsgemäße Mehrkomponenten-Verpackungseinheit dadurch gekennzeichnet, das Mittel (A) in Container (i) - bezogen auf das Gesamtgewicht des Mittels (A) - ein oder mehrere Keratin reduzierende Verbindungen in einer Gesamtmenge von 1,5 bis 20,0 Gew.-%, bevorzugt 3,0 bis 15,0 Gew.-%, weiter bevorzugt von 4,0 bis 12,0 Gew.-% und besonders bevorzugt von 4,5 bis 12,5 Gew.-% enthält.

### Bunte-Salze der Formel (I) in Mittel (B)

Das Mittel (B), welches im Container (ii) konfektioniert vorliegt, enthält als erfindungswesentlichen Inhaltsstoff mindestens ein Bunte-Salz einer Aminosäure, eines Oligopeptids oder eines Peptids.

Das Bunte-Salz einer Aminosäure, eines Oligopeptids oder eines Peptids wird durch die Formel (I) beschrieben 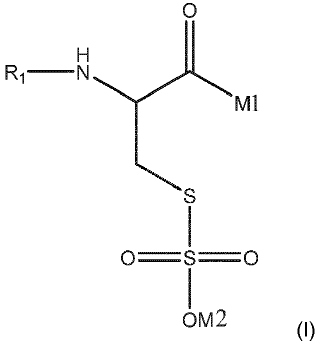 wobei
R1 steht für ein Wasserstoffatom oder für ein Strukturelement der Formel (II) 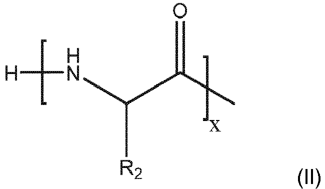 wobei
x für eine ganze Zahl von 1 bis 100 steht,
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 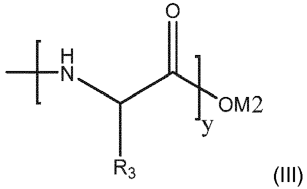
wobei
y für eine ganze Zahl von 1 bis 100 steht
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺.

Der Rest R1 kann entweder für ein Wasserstoffatom oder für ein Strukturelement der Formel (II) stehen

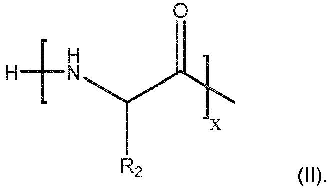

Das Strukturelement der Formel (II) ist weiterhin gekennzeichnet durch den Wiederholungsindex x, wobei x für eine ganze Zahl von 1 bis 100 steht. Der Wiederholungsindex x gibt an, wie viele Strukturelemente der Formel (II) in der Verbindung der Formel (I) enthalten sind.

Bevorzugt steht x für eine ganze Zahl von 1 bis 50, weiter bevorzugt steht x für eine ganze Zahl von 1 bis 20, und ganz besonders bevorzugt steht x für eine ganze Zahl von 1 bis 10.

Wenn x beispielweise für die Zahl 10 steht, beinhaltet die Verbindung der Formel (1) 10 Strukturelemente der Formel (II).

Hierbei ist wesentlich, dass der Rest R2 in jedem der Strukturelemente der Formel (II) jeweils unabhängig vom vorangegangenen Strukturelement der Formel (II) gewählt werden kann. Enthalten die Verbindungen der Formel (I) beispielsweise 10 Struktureinheiten der Formel (II), so können diese 10 Struktureinheiten gleich oder aber auch verschieden sein.

Der Rest R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethylGruppe, eine 1H-Imidazol-4-ylmethyl-Gruppe, eine 1H-Indol-3-ylmethyl-Gruppe oder für eine (Sulfosulfanyl)methyl-Gruppe.

Wenn x für die Zahl 1 steht, beinhaltet die Verbindung der Formel (I) ein Strukturelement der Formel (II). Hierbei erfolgt die Verknüpfung der Struktureinheiten wie in Formel (I-a) dargestellt.

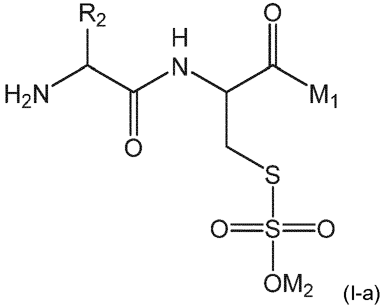

Wenn x für die Zahl 2 steht, beinhaltet die Verbindung der Formel (I) zwei Strukturelemente der Formel (II). Hierbei erfolgt die Verknüpfung der Struktureinheiten wie in Formel (I-b) dargestellt.

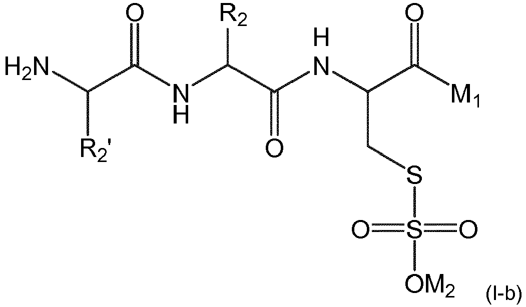

Hierbei kann, wie zuvor beschrieben, jeder der Reste R2 unabhängig vom anderen Rest R2 gewählt werden. Die unabhängig voneinander wählbaren Reste R2 sind in Formel (I-b) durch R2 und R2' gekennzeichnet.

Wenn x für die Zahl 3 steht, beinhaltet die Verbindung der Formel (I) drei Strukturelemente der Formel (II). Hierbei erfolgt die Verknüpfung der Struktureinheiten wie in Formel (I-c) dargestellt.

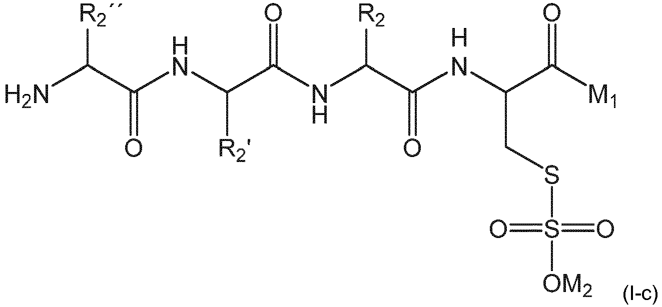

Hierbei kann, wie zuvor beschrieben, jeder der Reste R2 unabhängig vom anderen Rest R2 gewählt werden. Die unabhängig voneinander wählbaren Reste R2 sind in Formel (I-c) durch R2, R2' und R2" gekennzeichnet.

Wenn x für die Zahl 4 steht, beinhaltet die Verbindung der Formel (I) vier Strukturelemente der Formel (II). Hierbei erfolgt die Verknüpfung der Struktureinheiten wie in Formel (I-d) dargestellt. 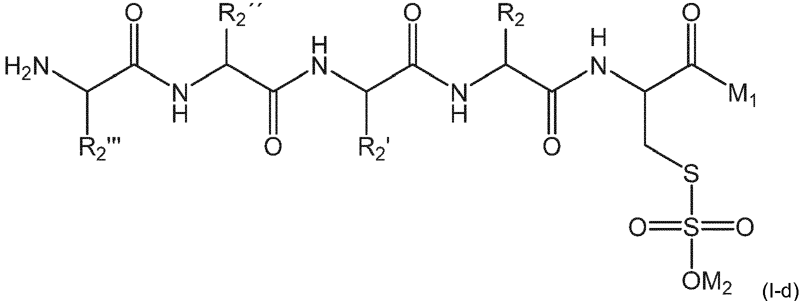 Hierbei kann, wie zuvor beschrieben, jeder der Reste R2 unabhängig von den anderen Resten R2 gewählt werden. Die unabhängig voneinander wählbaren Reste R2 sind in Formel (I-d) durch R2, R2', R2" und R2''' gekennzeichnet.

Wenn x für die Zahl 5 steht, beinhaltet die Verbindung der Formel (I) fünf Strukturelemente der Formel (II). Hierbei erfolgt die Verknüpfung der Struktureinheiten wie in Formel (I-e) dargestellt. 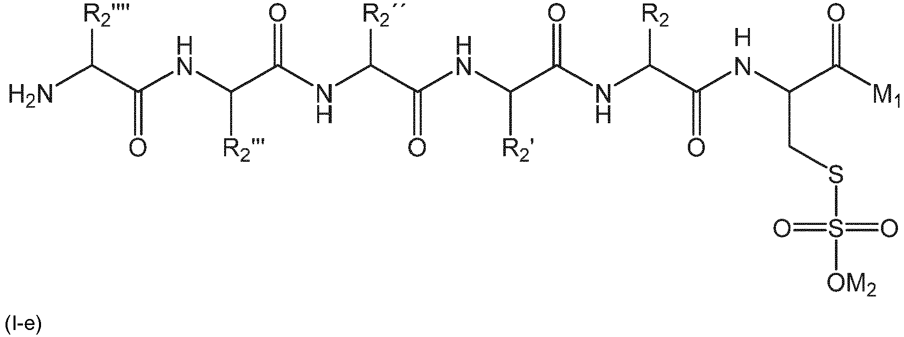 Hierbei kann, wie zuvor beschrieben, jeder der Reste R2 unabhängig von den anderen Resten R2 gewählt werden. Die unabhängig voneinander wählbaren Reste R2 sind in Formel (I-e) durch R2, R2', R2", R2''' und R2"" gekennzeichnet.

Wenn x für die Zahl 6 steht, beinhaltet die Verbindung der Formel (I) sechs Strukturelemente der Formel (II). Hierbei erfolgt die Verknüpfung der Struktureinheiten wie in Formel (I-f) dargestellt. 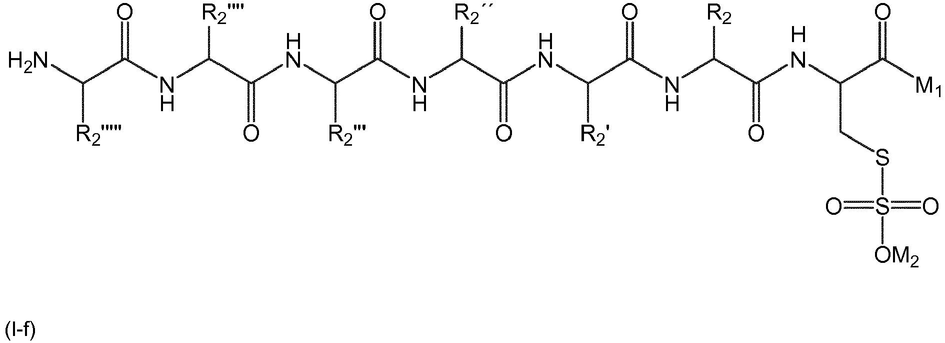 Hierbei kann, wie zuvor beschrieben, jeder der Reste R2 unabhängig von den anderen Resten R2 gewählt werden. Die unabhängig voneinander wählbaren Reste R2 sind in Formel (1-f) durch R2, R2', R2",R2"', R2"" und R2''''' gekennzeichnet.

Die Verknüpfung aller weiteren x Struktureinheiten erfolgt analog.

Bei dem Strukturelement der Formel (II) handelt es sich somit um eine Aminosäure, welche peptidisch über ihre Amino- und/oder ihre Säurefunktion innerhalb der Verbindung der Formel (I) verknüpft ist. Wenn es sich bei der Aminosäure um Cystein handelt, kann diese auch in Form eines Bunte-Salzes vorliegen.

Wenn der Rest R2 für ein Wasserstoffatom steht, so beruht das Strukturelement der Formel (II) auf der Aminosäure Glycin.
Wenn der Rest R2 für eine Methylgruppe steht, so beruht das Strukturelement der Formel (II) auf der Aminosäure Alanin.
Wenn der Rest R2 für eine Isopropylgruppe (d.h. eine Gruppe (H₃C)₂CH-) steht, so beruht das Strukturelement der Formel (II) auf der Aminosäure Valin.
Wenn der Rest R2 für eine 2-Methylpropylgruppe (d.h. eine Gruppe (H₃C)₂CH-CH₂-), so beruht das Strukturelement der Formel (II) auf der Aminosäure Leucin.
Wenn der Rest R2 für eine 1-Methyl-propylgruppe (d.h. eine Gruppe H3C-CH2-CH(CH3)-) steht, so beruht das Strukturelement der Formel (II) auf der Aminosäure Isoleucin.
Wenn der Rest R2 für eine Benzylgruppe (d.h. eine Gruppe C₆H₅-CH₂-) steht, so beruht das Strukturelement der Formel (II) auf der Aminosäure Phenylalanin.
Wenn der Rest R2 für eine 4-Hydroxybenzylgruppe (d.h. eine Gruppe 4-OH-C₆H₅-CH₂-) steht, so beruht das Strukturelement der Formel (II) auf der Aminosäure Tyrosin.
Wenn der Rest R2 für eine Hydroxymethylgruppe (d.h. eine Gruppe HO-CH2-) steht, so beruht das Strukturelement der Formel (II) auf der Aminosäure Serin.
Wenn der Rest R2 für eine 1-Hydroxyethylgruppe (d.h. eine Gruppe H3C-CH(OH)-) steht, so beruht das Strukturelement der Formel (II) auf der Aminosäure Threonin.
Wenn der Rest R2 für eine 4-Aminobutylgruppe (d.h. eine Gruppe H2N-CH2-CH2-CH2-CH2-) steht, so beruht das Strukturelement der Formel (II) auf der Aminosäure Lysin.
Wenn der Rest R2 für eine 3-Carbamimidamidopropyl-Gruppe (d.h. eine Gruppe H₂N-C(NH)-NH-CH₂-CH₂-CH₂-) steht, so beruht das Strukturelement der Formel (II) auf der Aminosäure Arginin.
Wenn der Rest R2 für eine 2-Carboxyethyl-Gruppe (d.h. eine Gruppe HOOC-CH2-CH2-) steht, so beruht das Strukturelement der Formel (II) auf der Aminosäure Glutaminsäure.
Wenn der Rest R2 für eine Carboxymethyl-Gruppe (d.h. eine Gruppe HOOC-CH2-) steht, so beruht das Strukturelement der Formel (II) auf der Aminosäure Asparaginsäure.
Wenn der Rest R2 für eine 2-Carbamoylethyl-Gruppe (d.h. eine Gruppe H2N-C(O)-CH2-CH2-) steht, so beruht das Strukturelement der Formel (II) auf der Aminosäure Glutamin.
Wenn der Rest R2 für eine Carbamoylmethyl-Gruppe (d.h. eine Gruppe H2N-C(O)-CH2-) steht, so beruht das Strukturelement der Formel (II) auf der Aminosäure Asparagin.
Wenn der Rest R2 für eine Sulfanylmethyl-Gruppe (d.h. eine Gruppe HS-CH2-) steht, so beruht das Strukturelement der Formel (II) auf der Aminosäure Cystein.
Wenn der Rest R2 für eine 2-(Methylsulfanyl)ethyl-Gruppe (d.h. eine Gruppe H3C-S-CH2-CH2-) steht, so beruht das Strukturelement der Formel (II) auf der Aminosäure Methionin.
Wenn der Rest R2 für eine 1H-Imidazol-4-ylmethyl-Gruppe steht, so beruht das Strukturelement der Formel (II) auf der Aminosäure Histidin.
Wenn der Rest R2 für eine 1H-Indol-3-ylmethyl-Gruppe steht, so beruht das Strukturelement der Formel (II) auf der Aminosäure Tryptophan.
Schließlich kann der Rest R2 auch für eine (Sulfosulfanyl)methyl-Gruppe stehen, hierbei handelt es sich um eine Bunte-Salz Struktur der Formel HO-S(O₂)-S-CH₂-.

Je nach pH-Wert des Haarverformungsmittels kann die Bunte-Salz Struktur der Formel HO-S(O₂)-S-CH₂- auch in ihrer deprotonierten Form vorliegen.

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-a) enthält, 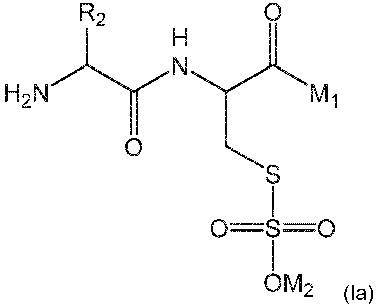 wobei
   R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   M1 steht für die Gruppierung -OM2 oder für ein Strukturelement der Formel (III) 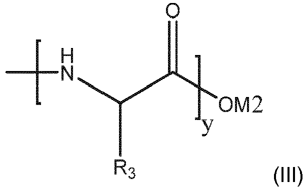
   wobei
   y für eine ganze Zahl von 1 bis 10 steht,
   der Rest R3 in jedem der Strukturelemente der Formel (III) jeweils unabhängig vom vorangegangenen Strukturelement der Formel (III) gewählt werden kann,
   R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-b) enthält, 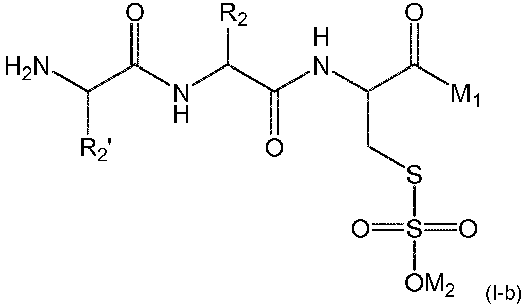 wobei
   R2 und R2' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   M1 steht für die Gruppierung -OM2 oder für ein Strukturelement der Formel (III) 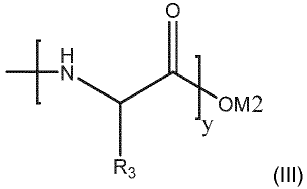
   wobei
   y für eine ganze Zahl von 1 bis 10 steht,
   der Rest R3 in jedem der Strukturelemente der Formel (III) jeweils unabhängig vom vorangegangenen Strukturelement der Formel (III) gewählt werden kann,
   R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-c) enthält, 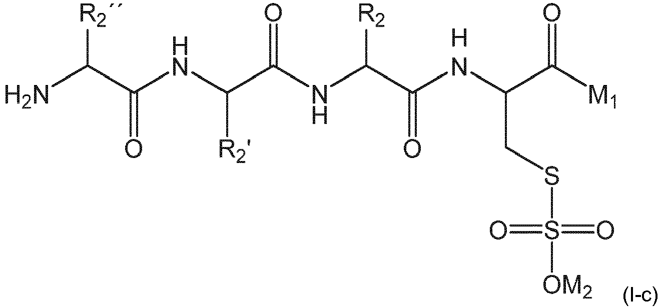 wobei
   R2, R2' und R2" unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   M1 steht für die Gruppierung -OM2 oder für ein Strukturelement der Formel (III) 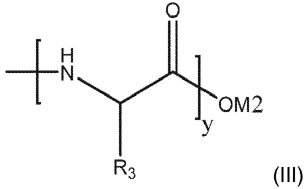
   wobei
   y für eine ganze Zahl von 1 bis 10 steht,
   der Rest R3 in jedem der Strukturelemente der Formel (III) jeweils unabhängig vom vorangegangenen Strukturelement der Formel (III) gewählt werden kann,
   R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺ und

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-d) enthält, 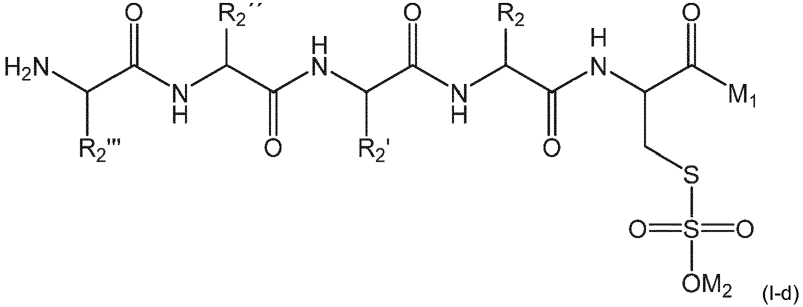 wobei
   R2, R2', R2" und R2''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1 H-Indol-3-ylmethyl-Gruppe oder eine (Sulfosulfanyl)methyl-Gruppe,
   M1 steht für die Gruppierung -OM2 oder für ein Strukturelement der Formel (III) 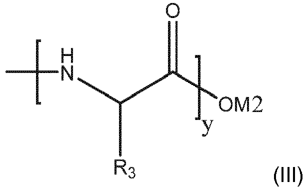
   wobei
   y für eine ganze Zahl von 1 bis 10 steht,
   der Rest R3 in jedem der Strukturelemente der Formel (III) jeweils unabhängig vom vorangegangenen Strukturelement der Formel (III) gewählt werden kann,
   R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-e) enthält, 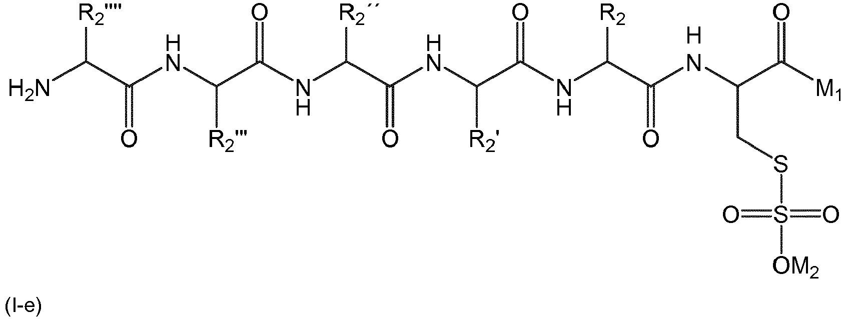 wobei
   R2, R2', R2", R2''' und R2'''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1 H-Indol-3-ylmethyl-Gruppe oder eine (Sulfosulfanyl)methyl-Gruppe,
   M1 steht für die Gruppierung -OM2 oder für ein Strukturelement der Formel (III) 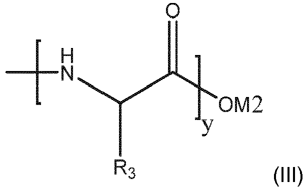
   wobei
   y für eine ganze Zahl von 1 bis 10 steht,
   der Rest R3 in jedem der Strukturelemente der Formel (III) jeweils unabhängig vom vorangegangenen Strukturelement der Formel (III) gewählt werden kann,
   R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-f) enthält, 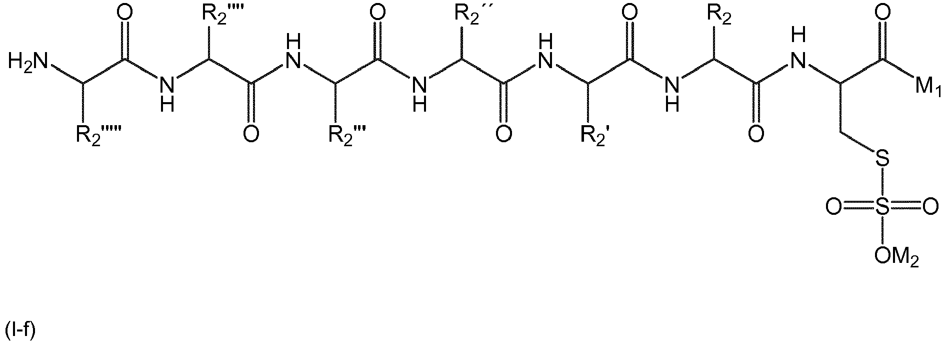 wobei
   R2, R2', R2", R2''', R2"" und R2''''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1 H-Indol-3-ylmethyl-Gruppe oder eine (Sulfosulfanyl)methyl-Gruppe,
   M1 steht für die Gruppierung -OM2 oder für ein Strukturelement der Formel (III) 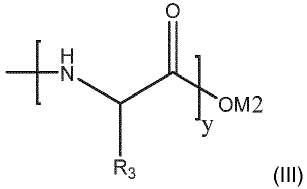
   wobei
   y für eine ganze Zahl von 1 bis 10 steht,
   der Rest R3 in jedem der Strukturelemente der Formel (III) jeweils unabhängig vom vorangegangenen Strukturelement der Formel (III) gewählt werden kann,
   R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺.

Innerhalb der Verbindung der Formel (I) steht M1 steht die Gruppierung -OM2 oder für ein Strukturelement der Formel (III)

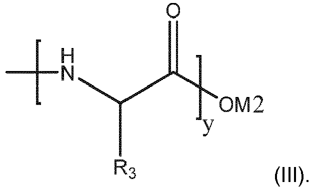

Das Strukturelement der Formel (III) ist - genau wie das Strukturelement der Formel (II) - durch einen Wiederholungsindex gekennzeichnet. Der Wiederholungsindex y steht für eine ganze Zahl von 1 bis 100. Der Wiederholungsindex y gibt an, wie viele Strukturelemente der Formel (III) in der Verbindung der Formel (I) enthalten sind.

Bevorzugt steht y für eine ganze Zahl von 1 bis 50, weiter bevorzugt steht y für eine ganze Zahl von 1 bis 20, und ganz besonders bevorzugt steht y für eine ganze Zahl von 1 bis 10.

Wenn y beispielweise für die Zahl 10 steht, beinhaltet die Verbindung der Formel (I) 10 Strukturelemente der Formel (III).

Hierbei ist wesentlich, dass der Rest R3 in jedem der Strukturelemente der Formel (III) jeweils unabhängig vom vorangegangenen Strukturelement der Formel (III) gewählt werden kann. Enthalten die Verbindungen der Formel (I) beispielsweise 10 Struktureinheiten der Formel (III), so können diese 10 Struktureinheiten gleich oder aber auch verschieden sein.

Der Rest R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethylGruppe, eine 1H-Imidazol-4-ylmethyl-Gruppe, eine 1H-Indol-3-ylmethyl-Gruppe oder eine (Sulfosulfanyl)methyl-Gruppe.

Damit handelt es sich auch bei dem Strukturelement der Formel (III) um eine Aminosäure, welche peptidisch über ihre Amino- und/oder ihre Säurefunktion innerhalb der Verbindung der Formel (I) verknüpft ist. Wenn es sich bei der Aminosäure um Cystein handelt, kann diese auch in Form eines Bunte-Salzes vorliegen.

Wenn y für die Zahl 1 steht, beinhaltet die Verbindung der Formel (I) ein Strukturelement der Formel (III). Hierbei erfolgt die Verknüpfung der Struktureinheiten wie in Formel (I-I) dargestellt.

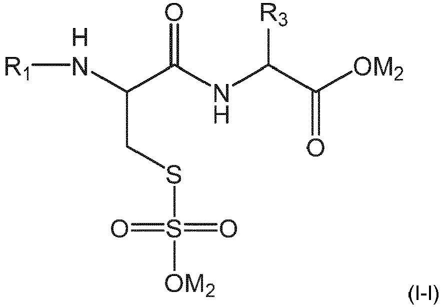

Wenn y für die Zahl 2 steht, beinhaltet die Verbindung der Formel (I) zwei Strukturelemente der Formel (III). Hierbei erfolgt die Verknüpfung der Struktureinheiten wie in Formel (I-II) dargestellt.

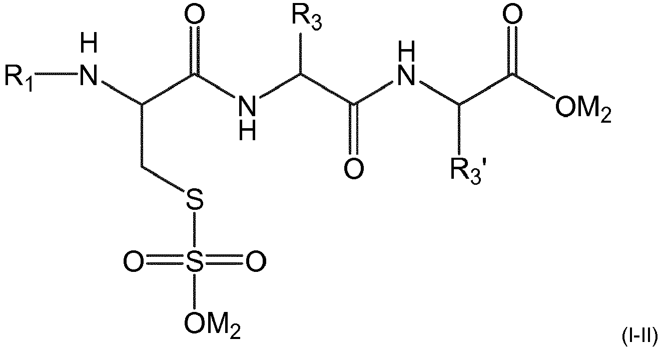

Hierbei kann, wie zuvor beschrieben, jeder der Reste R3 unabhängig vom anderen Rest R3 gewählt werden. Die unabhängig voneinander wählbaren Reste R3 sind in Formel (I-II) durch R3 und R3' gekennzeichnet.

Wenn y für die Zahl 3 steht, beinhaltet die Verbindung der Formel (I) drei Strukturelemente der Formel (III). Hierbei erfolgt die Verknüpfung der Struktureinheiten wie in Formel (I-III) dargestellt.

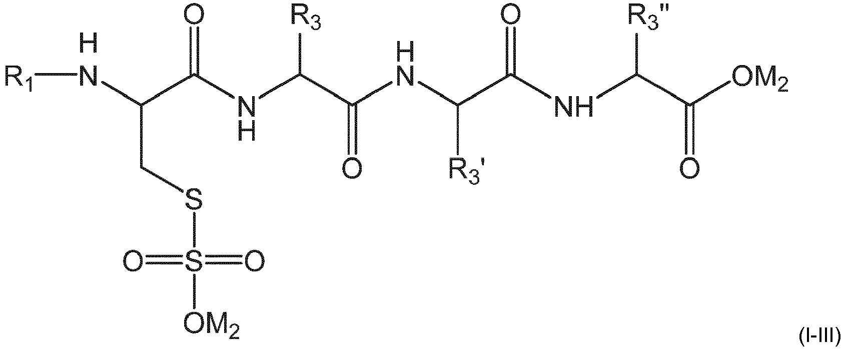

Hierbei kann, wie zuvor beschrieben, jeder der Reste R3 unabhängig von den anderen Resten R3 gewählt werden. Die unabhängig voneinander wählbaren Reste R3 sind in Formel (I-III) durch R3, R3' und R3" gekennzeichnet.

Wenn y für die Zahl 4 steht, beinhaltet die Verbindung der Formel (I) vier Strukturelemente der Formel (III). Hierbei erfolgt die Verknüpfung der Struktureinheiten wie in Formel (I-IV) dargestellt. 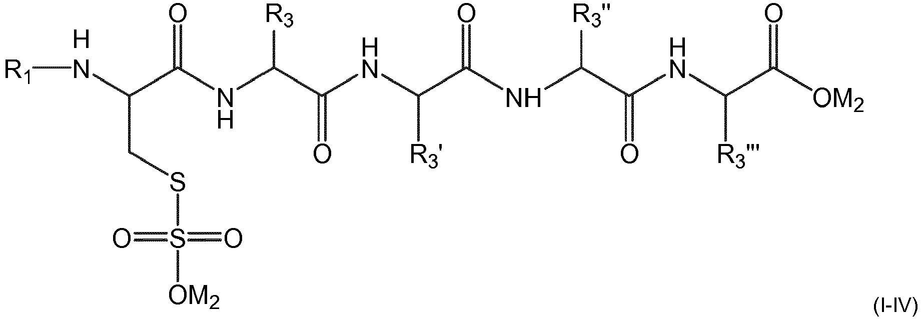 Hierbei kann, wie zuvor beschrieben, jeder der Reste R3 unabhängig von den anderen Resten R3 gewählt werden. Die unabhängig voneinander wählbaren Reste R3 sind in Formel (I-IV) durch R3, R3', R3" und R3''' gekennzeichnet.

Wenn y für die Zahl 5 steht, beinhaltet die Verbindung der Formel (I) fünf Strukturelemente der Formel (II). Hierbei erfolgt die Verknüpfung der Struktureinheiten wie in Formel (I-V) dargestellt. 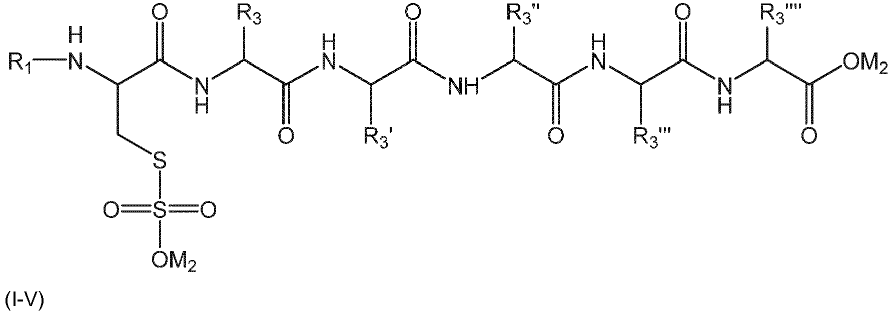 Hierbei kann, wie zuvor beschrieben, jeder der Reste R3 unabhängig von den anderen Resten R3 gewählt werden. Die unabhängig voneinander wählbaren Reste R3 sind in Formel (I-e) durch R3, R3', R3'',R3''' und R3"" gekennzeichnet.

Wenn y für die Zahl 6 steht, beinhaltet die Verbindung der Formel (I) sechs Strukturelemente der Formel (II). Hierbei erfolgt die Verknüpfung der Struktureinheiten wie in Formel (I-VI) dargestellt. 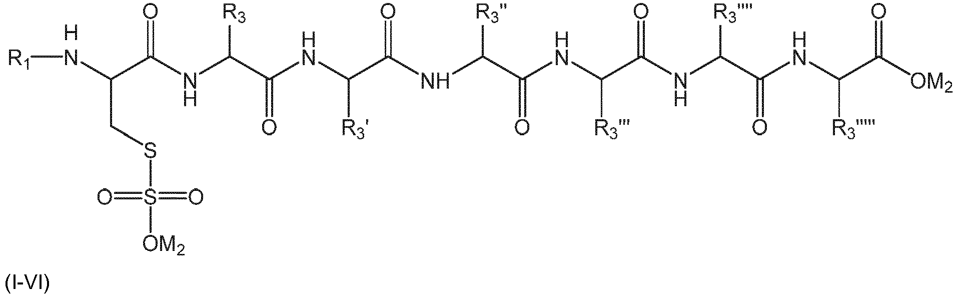 Hierbei kann, wie zuvor beschrieben, jeder der Reste R3 unabhängig von den anderen Resten R3 gewählt werden. Die unabhängig voneinander wählbaren Reste R3 sind in Formel (I-VI) durch R3, R3', R3", R3''', R3"" und R3''''' gekennzeichnet.

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-I) enthält, 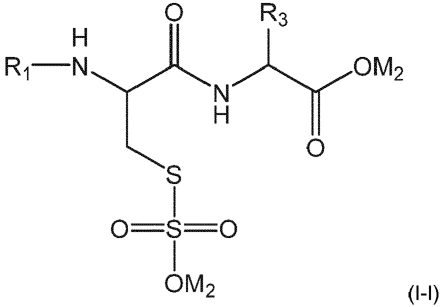 wobei
   R1 steht für ein Wasserstoffatom oder für ein Strukturelement der Formel (II) 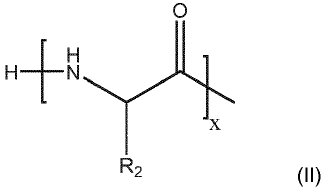 wobei
   x für eine ganze Zahl von 1 bis 10 steht
   der Rest R2 in jedem der Strukturelemente der Formel (II) jeweils unabhängig vom vorangegangenen Strukturelement der Formel (II) gewählt werden kann,
   R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-II) enthält, 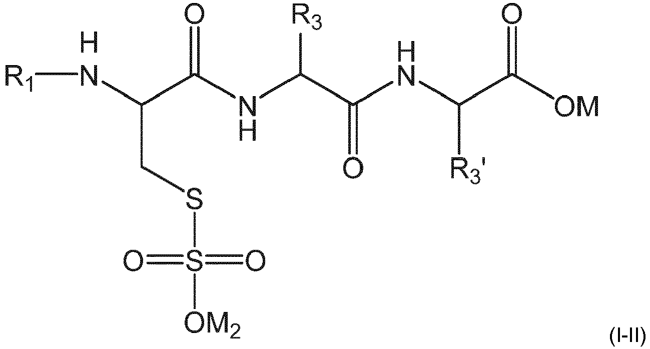 wobei
   R1 steht für ein Wasserstoffatom oder für ein Strukturelement der Formel (II) 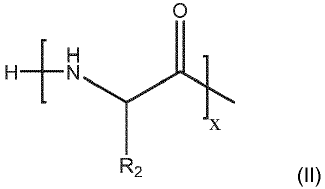 wobei
   x für eine ganze Zahl von 1 bis 10 steht
   der Rest R2 in jedem der Strukturelemente der Formel (II) jeweils unabhängig vom vorangegangenen Strukturelement der Formel (II) gewählt werden kann,
   R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   R3 und R3' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-III) enthält, 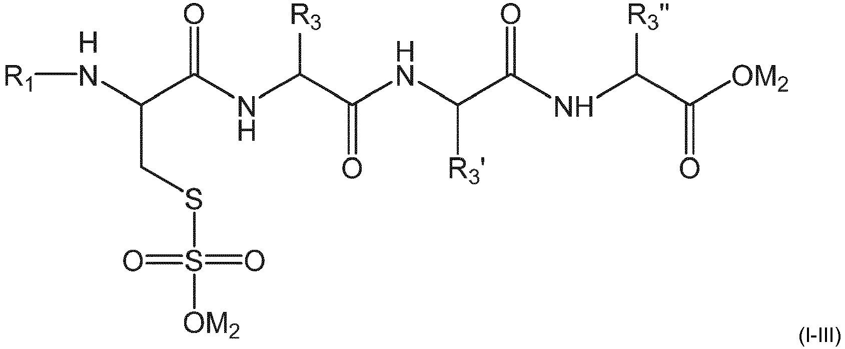 wobei
   R1 steht für ein Wasserstoffatom oder für ein Strukturelement der Formel (II) 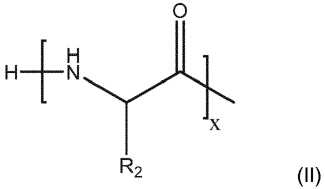 wobei
   x für eine ganze Zahl von 1 bis 10 steht
   der Rest R2 in jedem der Strukturelemente der Formel (II) jeweils unabhängig vom vorangegangenen Strukturelement der Formel (II) gewählt werden kann,
   R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   R3, R3' und R3" unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1 H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-IV) enthält, 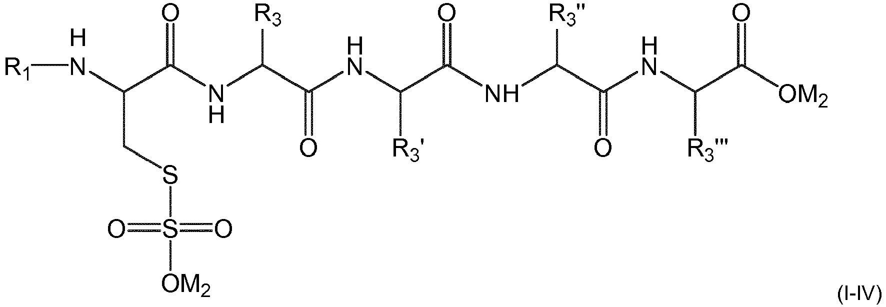 wobei
   R1 steht für ein Wasserstoffatom oder für ein Strukturelement der Formel (II) 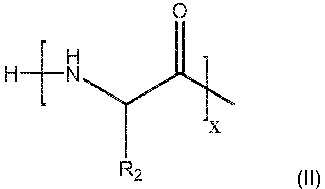 wobei
   x für eine ganze Zahl von 1 bis 10 steht
   der Rest R2 in jedem der Strukturelemente der Formel (II) jeweils unabhängig vom vorangegangenen Strukturelement der Formel (II) gewählt werden kann,
   R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   R3, R3', R3" und R3''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-V) enthält, 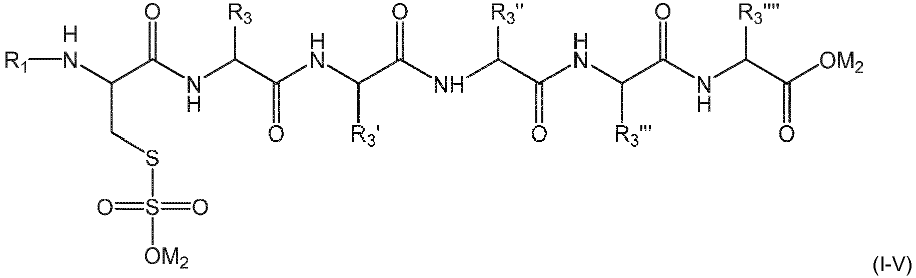 wobei
   R1 steht für ein Wasserstoffatom oder für ein Strukturelement der Formel (II) 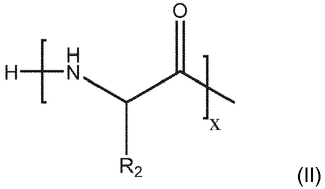 wobei
   x für eine ganze Zahl von 1 bis 10 steht
   der Rest R2 in jedem der Strukturelemente der Formel (II) jeweils unabhängig vom vorangegangenen Strukturelement der Formel (II) gewählt werden kann,
   R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   R3, R3', R3", R3''' und R3"" unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass
- das Mittel (B) in Container (ii) mindestens eine Verbindung der allgemeinen Formel (I-VI) enthält, 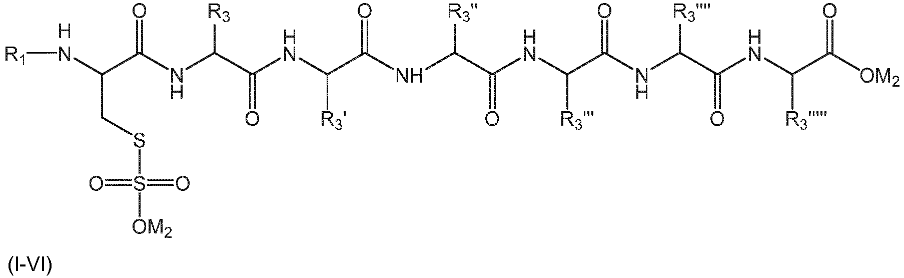 wobei
   R1 steht für ein Wasserstoffatom oder für ein Strukturelement der Formel (II) 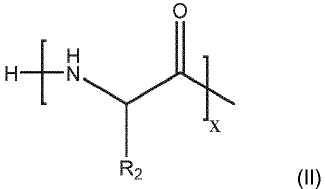 wobei
   x für eine ganze Zahl von 1 bis 10 steht
   der Rest R2 in jedem der Strukturelemente der Formel (II) jeweils unabhängig vom vorangegangenen Strukturelement der Formel (II) gewählt werden kann,
   R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   R3, R3', R3", R3''', R3"" und R3''''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1 H-Indol-3-ylmethyl-Gruppe oder eine (Sulfosulfanyl)methyl-Gruppe, und
   M2 steht für ein Wasserstoffatom, ein Äquivalent eines ein oder mehrwertigen Kations oder ein Ammoniumion (NH₄)⁺.

Wenn der Rest R3 für ein Wasserstoffatom steht, so beruht das Strukturelement der Formel (II) auf der Aminosäure Glycin.
Wenn der Rest R3 für eine Methylgruppe steht, so beruht das Strukturelement der Formel (II) auf der Aminosäure Alanin.
Wenn der Rest R3 für eine Isopropylgruppe (d.h. eine Gruppe (H₃C)₂CH-) steht, so beruht das Strukturelement der Formel (II) auf der Aminosäure Valin.
Wenn der Rest R3 für eine 2-Methylpropylgruppe (d.h. eine Gruppe (H₃C)₂CH-CH₂-), so beruht das Strukturelement der Formel (II) auf der Aminosäure Leucin.
Wenn der Rest R3 für eine 1-Methyl-propylgruppe (d.h. eine Gruppe H3C-CH2-CH(CH3)-) steht, so beruht das Strukturelement der Formel (II) auf der Aminosäure Isoleucin.
Wenn der Rest R3 für eine Benzylgruppe (d.h. eine Gruppe C₆H₅-CH₂-) steht, so beruht das Strukturelement der Formel (II) auf der Aminosäure Phenylalanin.
Wenn der Rest R3 für eine 4-Hydroxybenzylgruppe (d.h. eine Gruppe 4OH-C₆H₅-CH₂-) steht, so beruht das Strukturelement der Formel (II) auf der Aminosäure Tyrosin.
Wenn der Rest R3 für eine Hydroxymethylgruppe (d.h. eine Gruppe HO-CH2-) steht, so beruht das Strukturelement der Formel (II) auf der Aminosäure Serin.
Wenn der Rest R3 für eine 1-Hydroxyethylgruppe (d.h. eine Gruppe H3C-CH(OH)-) steht, so beruht das Strukturelement der Formel (II) auf der Aminosäure Threonin.
Wenn der Rest R3 für eine 4-Aminobutylgruppe (d.h. eine Gruppe H2N-CH2-CH2-CH2-CH2-) steht, so beruht das Strukturelement der Formel (II) auf der Aminosäure Lysin.
Wenn der Rest R3 für eine 3-Carbamimidamidopropyl-Gruppe (d.h. eine Gruppe H₂N-C(NH)-NH-CH₂-CH₂-CH₂-) steht, so beruht das Strukturelement der Formel (II) auf der Aminosäure Arginin.
Wenn der Rest R3 für eine 2-Carboxyethyl-Gruppe (d.h. eine Gruppe HOOC-CH2-CH2-) steht, so beruht das Strukturelement der Formel (II) auf der Aminosäure Glutaminsäure.
Wenn der Rest R3 für eine Carboxymethyl-Gruppe (d.h. eine Gruppe HOOC-CH2-) steht, so beruht das Strukturelement der Formel (II) auf der Aminosäure Asparaginsäure.
Wenn der Rest R3 für eine 2-Carbamoylethyl-Gruppe (d.h. eine Gruppe H2N-C(O)-CH2-CH2-) steht, so beruht das Strukturelement der Formel (II) auf der Aminosäure Glutamin.
Wenn der Rest R3 für eine Carbamoylmethyl-Gruppe (d.h. eine Gruppe H2N-C(O)-CH2-) steht, so beruht das Strukturelement der Formel (II) auf der Aminosäure Asparagin.
Wenn der Rest R3 für eine Sulfanylmethyl-Gruppe (d.h. eine Gruppe HS-CH2-) steht, so beruht das Strukturelement der Formel (II) auf der Aminosäure Cystein.
Wenn der Rest R3 für eine 2-(Methylsulfanyl)ethyl-Gruppe (d.h. eine Gruppe H3C-S-CH2-CH2-) steht, so beruht das Strukturelement der Formel (II) auf der Aminosäure Methionin.
Wenn der Rest R3 für eine 1H-Imidazol-4-ylmethyl-Gruppe steht, so beruht das Strukturelement der Formel (II) auf der Aminosäure Histidin.
Wenn der Rest R3 für eine 1H-Indol-3-ylmethyl-Gruppe steht, so beruht das Strukturelement der Formel (II) auf der Aminosäure Tryptophan.
Schließlich kann der Rest R3 auch für eine (Sulfosulfanyl)methyl-Gruppe stehen, hierbei handelt es sich um eine Bunte-Salz Struktur der Formel HO-S(O₂)-S-CH₂-.

Je nach pH-Wert des Haarverformungsmittels kann auch hier die Bunte-Salz Struktur der Formel HO-S(O₂)-S-CH₂- auch in ihrer deprotonierten Form vorliegen.

Der Rest M2 steht für ein Wasserstoffatom, ein Äquivalent eines ein oder mehrwertigen Kations oder ein Ammoniumion (NH₄)⁺.

Als bevorzugte Äquivalente eines ein oder mehrwertigen Kations können insbesondere die Kationen von Natrium und Kalium (Na⁺ bzw. K⁺) oder auch Magnesium oder Caliucm (1/2 Mg2+ oder ½ Ca2+) genannt werden.

Steht M2 für ein Wasserstoffatom, so handelt es sich bei der Gruppierung -OM2 um die Gruppierung - OH. Steht M2 für ein Natriumkation, so handelt es sich bei der Gruppierung -OM2 um die Gruppierung -ONa. Steht M2 für ein Kaliumkation, so handelt es sich bei der Gruppierung -OM2 um die Gruppierung -OK. Steht M2 für ein Ammoniumion, so handelt es sich bei der Gruppierung -OM2 um die Gruppierung -O(NH₄).

Bei den erfindungsgemäßen Verbindungen der Formel (I) handelt es sich entweder um das Bunte-Salz der Aminosäure Cystein, um die Bunte-Salze von Oligo-Peptiden oder aber um die Bunte-Salze von Peptiden.

Wenn der Rest R1 für ein Wasserstoffatom steht und der Rest M1 für eine Gruppierung -OM2 steht, dann handelt es sich bei der Verbindung der Formel (I) um das Bunte-Salz der Aminosäure Cystein. In diesem Fall handelt es sich bei der Verbindung der Formel (I) um die Verbindung der Formel (IA), 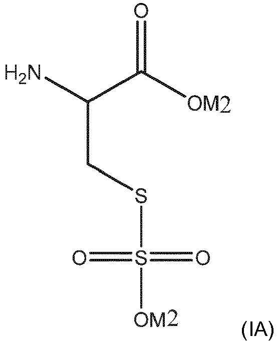 wobei M2 wieder wie zuvor beschrieben definiert wird.

Liegt die Verbindung der Formel (IA) in Form ihrer freien Säure vor, so handelt es sich um 2-Amino-3-(sulfosulfanyl)propansäure. Diese Substanz ist kommerziell erhältlich.

Es hat sich herausgestellt, dass der Einsatz der Verbindung der Formel (IA) in Haarverformungsmittel bereits bei besonders geringen Einsatzmengen zu einer besonders effektiven Reduzierung der Haarschädigung führt, die auch nach wiederholten Haarwäschen noch vorhanden ist. Deshalb ist der Einsatz von Verbindungen der Formel (IA) in Haarverformungsmitteln ganz besonders bevorzugt.

In einer ganz besonders bevorzugten Ausführungsform ist eine erfindungsgemäße Mehrkomponenten-Verpackungseinheit nach Anspruch 1 bis 4, dadurch gekennzeichnet, dass das Mittel (B) in Container (ii) mindestens eine Verbindung der Formel (I) enthält, wobei
R1 für ein Wasserstoffatom steht und
M1 für eine Gruppierung -OM2 steht.

Wenn eine Verbindung der Formel (IA) eingesetzt wird, so handelt es sich bevorzugt um den Einsatz dieser spezifischen Verbindung. Werden als Verbindungen der Formel (I) jedoch die Bunte-Salze von Oligopeptiden eingesetzt, so kann das erfindungsgemäße Haarverformungsmittel auch mehrere Verbindungen der Formel (I) als Gemisch verschiedener Oligopeptide enthalten. Diese Oligopeptide werden durch ihr mittleres Molgewicht definiert. Das mittlere Molekulargewicht M_{w} des mindestens einen Oligopeptids der Formel (I) kann beispielsweise durch Gelpermeationschromatographie (GPC) mit Polystyrol als internem Standard gemäß DIN 55672-3 bestimmt werden.

Je nachdem, wie viele Strukturelemente der Formel (II) und/oder (III) in der Verbindung der Formel (I) enthalten sind, und je nach Art dieser Aminosäuren kann das Molgewicht der erfindungsgemäß verwendeten Verbindung der Formel (I) variieren. Es ist erfindungsgemäß besonders bevorzugt, wenn es sich bei der Verbindung der Formel (I) um ein Oligopeptid handelt, welches ein Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Unter dem Begriff "Oligopeptid" im Rahmen der vorliegenden Erfindung werden Kondensationsprodukte von Aminosäuren verstanden, welche die oben genannten Molekulargewichte besitzen.

In einer ganz besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I) enthält, die ein Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Wird im erfindungsgemäßen Haarverformungsmittel eine Mischung von Oligomeren eingesetzt, so können diese Mischungen durch ihr mittleres Molekulargewicht definiert werden.

In einer ganz besonders bevorzugten Ausführungsform ist eine erfindungsgemäße Mehrkomponenten-Verpackungseinheit dadurch gekennzeichnet, dass das Mittel (B) in Container (ii) Verbindungen der Formel (I) enthält, die ein mittleres Molekulargewicht M_{w} von 200 bis 2.000 Da, vorzugsweise von 250 bis 1.500 Da, bevorzugt von 300 bis 1.200 Da, insbesondere von 400 bis 800 Da aufweisen.

Weiterhin hat sich gezeigt, dass der Repair-Effekt, den die Verbindungen der Formel (I) besitzen, auch von den Wiederholungsindices x und y abhängt. Wie zuvor beschrieben, ist es ganz besonders bevorzugt, wenn x für eine ganze Zahl von 1 bis 10 steht und y für eine ganze Zahl von 1 bis 10 steht.

In einer weiteren ganz besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I) enthält, wobei
R1 für ein Strukturelement der Formel (II) steht, und
M1 für ein Strukturelement der Formel (III) steht, und
x für eine ganze Zahl von 1 bis 10 steht und
y für eine ganze Zahl von 1 bis 10 steht.

Neben dem Molekulargewicht der Verbindung der Formel (I) nimmt auch der Anteil der Bunte-Salz Einheiten, die in der Verbindung der Formel (I) enthalten sind, einen entscheidenden Einfluss auf die Wirksamkeit der "Repair-Wirkung" der Verbindungen.

Verbindungen mit mindestens einer Bunte-Salz-Einheit - wie sie beispielsweise in der Verbindung der Formel (la) vorhanden ist - sind sehr effektiv, insbesondere wenn sie als monomere Verbindung eingesetzt werden. Oligopeptide mit mindestens einer Bunte-Salz Einheit sind besonders wirksam, wenn sie ein niedriges Molekulargewicht von bis zu 1200, insbesondere 800 Dalton besitzen.

Bei Einsatz von Oligopeptiden ist es jedoch von ganz besonderem Vorteil, wenn die Verbindung der Formel (I) mindestens zwei, bevorzugt mindestens drei Bunte-Salz Einheiten besitzt.

In einer weiteren ganz besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I) enthält, wobei
R1 für ein Strukturelement der Formel (II) steht, und
der Rest R2 in mindestens einem Strukturelement der Formel (II) für eine (Sulfosulfanyl)methylGruppe (d.h. eine Gruppe HO-S(O₂)-S-CH₂-)steht.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-a') enthält 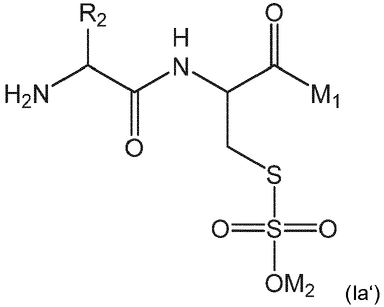 wobei
R2 steht für eine (Sulfosulfanyl)methyl-Gruppe (d.h. eine Gruppe HO-S(O₂)-S-CH₂-) und
M1 steht für die Gruppierung -OM2 oder für ein Strukturelement der Formel (III) 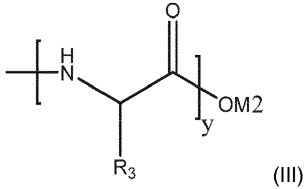
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-b') enthält 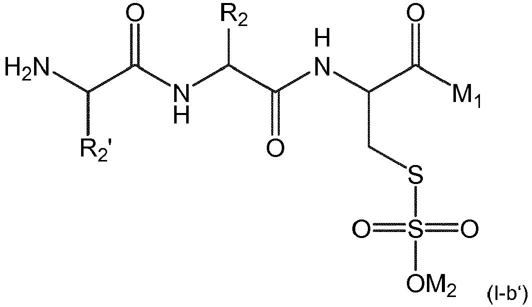 wobei
einer der Reste aus R2 und R2' steht für eine (Sulfosulfanyl)methyl-Gruppe (d.h. eine Gruppe HO-S(O₂)-S-CH₂-) und
der andere der Reste aus R2 und R2' steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 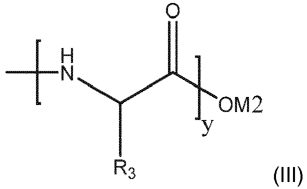
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-c') enthält 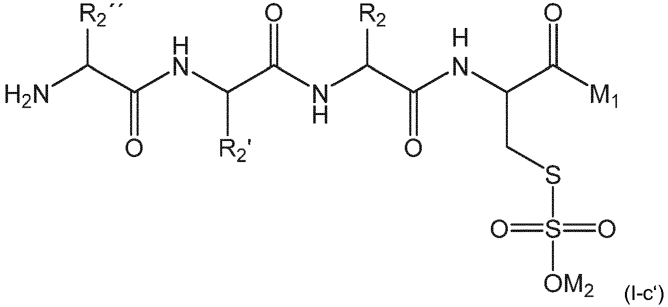 wobei
einer der Reste aus R2, R2' und R2" steht für eine (Sulfosulfanyl)methyl-Gruppe (d.h. eine Gruppe HO-S(O₂)-S-CH₂-) und
die anderen beiden Reste aus R2, R2' und R2" unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 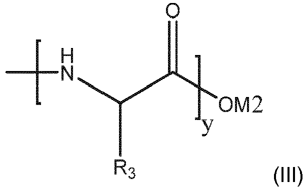
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-d') enthält 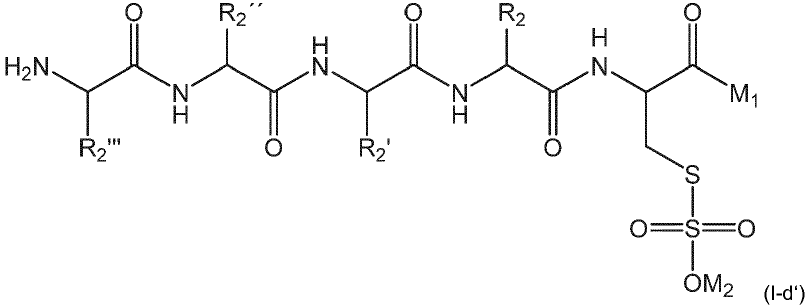 wobei
einer der Reste aus R2, R2', R2" und R2''' steht für eine (Sulfosulfanyl)methyl-Gruppe (d.h. eine Gruppe HO-S(O₂)-S-CH₂-) und
die anderen drei Reste aus R2, R2', R2" und R2''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 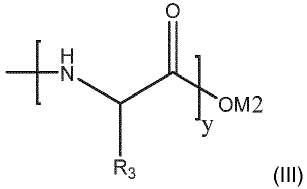
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-e') enthält 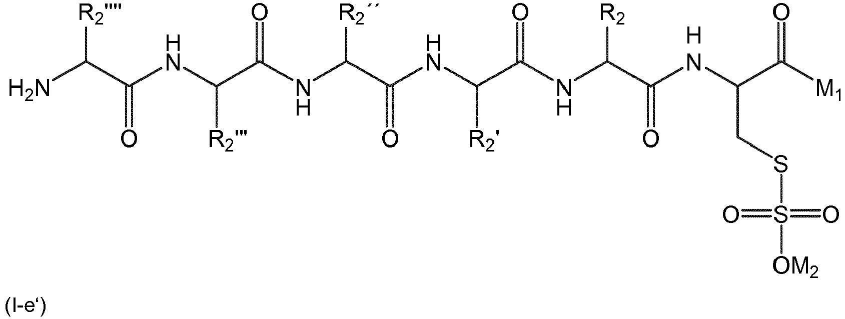 wobei
einer der Reste aus R2, R2', R2", R2''' und R2'''' steht für eine (Sulfosulfanyl)methyl-Gruppe (d.h. eine Gruppe HO-S(O₂)-S-CH₂-) und
die anderen vier Reste aus R2, R2', R2", R2''' und R2'''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 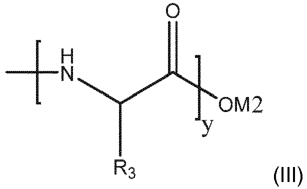
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-f') enthält 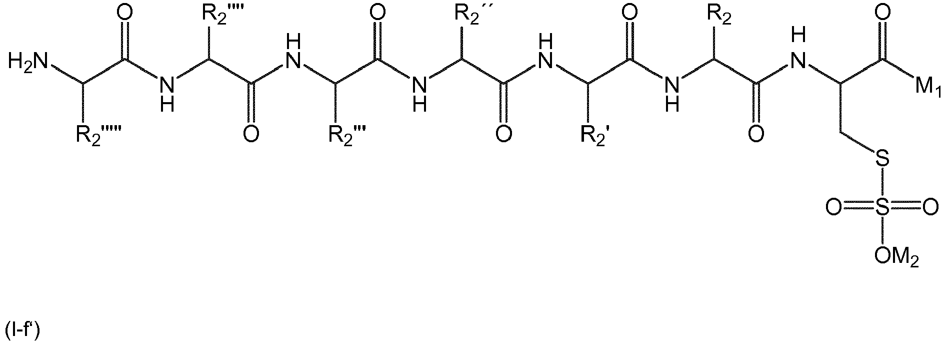 wobei
einer der Reste aus R2, R2', R2", R2"', R2'''' und R2''''' steht für eine (Sulfosulfanyl)methyl-Gruppe (d.h. eine Gruppe HO-S(O₂)-S-CH₂-) und
die anderen fünf Reste aus R2, R2', R2", R2''', R2"" und R2''''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 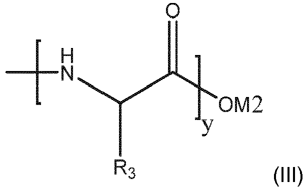
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren ganz besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I) enthält, wobei
R1 für ein Strukturelement der Formel (II) steht, und
x für eine ganze Zahl von mindestens 3 steht und
der Rest R2 in mindestens einem Strukturelement der Formel (II) für eine 2-Carboxyethyl-Gruppe steht.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-a'') enthält 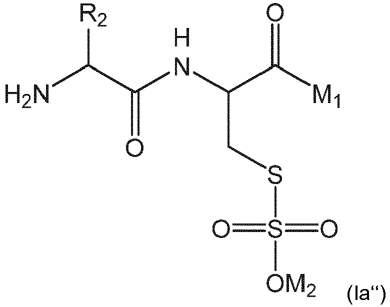 wobei
R2 steht für eine 2-Carboxyethyl-Gruppe (d.h. eine Gruppe HOOC-CH2-CH2-), und
M1 steht für die Gruppierung -OM2 oder für ein Strukturelement der Formel (III) 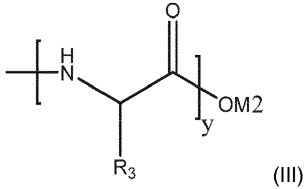
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-b'') enthält 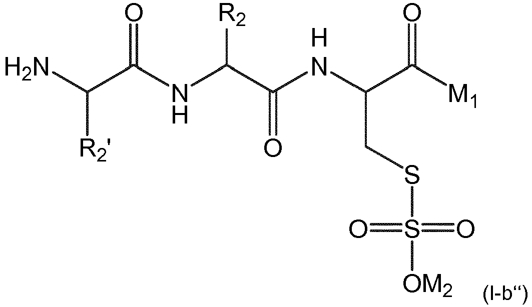 wobei
einer der Reste aus R2 und R2' steht für eine 2-Carboxyethyl-Gruppe (d.h. eine Gruppe HOOC-CH2-CH2-) und
der andere der Reste aus R2 und R2' steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 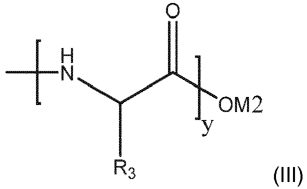
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-c") enthält 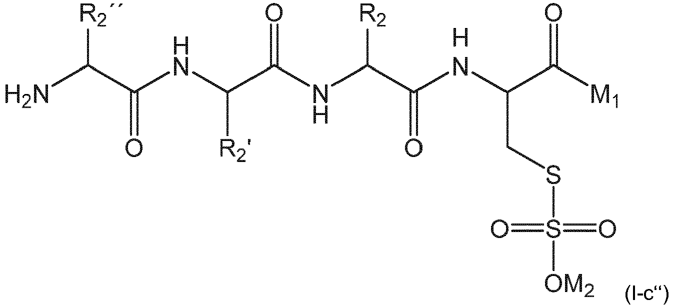 wobei
einer der Reste aus R2, R2' und R2" steht für eine 2-Carboxyethyl-Gruppe (d.h. eine Gruppe HOOC-CH2-CH2-) und
die anderen Reste aus R2, R2' und R2" unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1 H-Indol-3-ylmethyl-Gruppe oder eine (Sulfosulfanyl)methyl-Gruppe,
M1 steht für die Gruppierung -OM2 oder für ein Strukturelement der Formel (III) 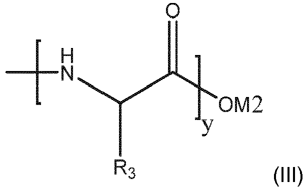
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-d") enthält 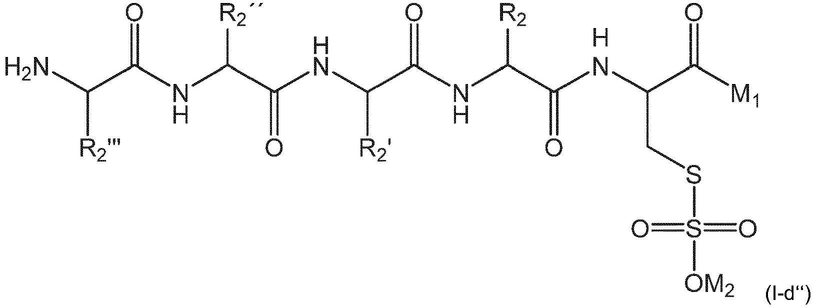 wobei
einer der Reste aus R2, R2', R2" und R2''' steht für eine 2-Carboxyethyl-Gruppe (d.h. eine Gruppe HOOC-CH2-CH2-) und
die anderen Reste aus R2, R2', R2" und R2''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 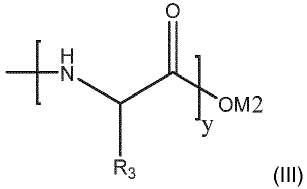
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-e") enthält 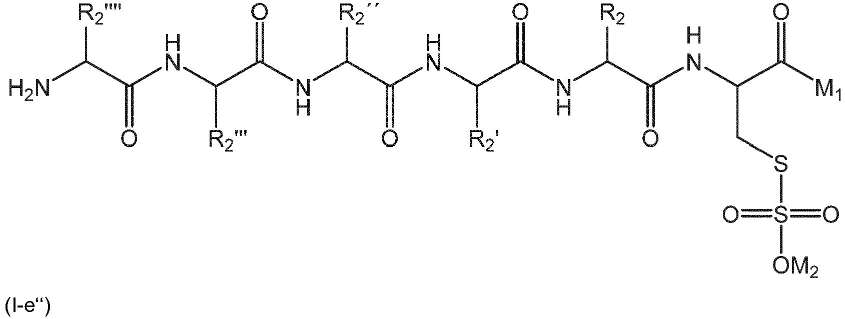 wobei
einer der Reste aus R2, R2', R2", R2''' und R2'''' steht für eine 2-Carboxyethyl-Gruppe (d.h. eine Gruppe HOOC-CH2-CH2-) und
die anderen Reste aus R2, R2', R2", R2''' und R2'''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 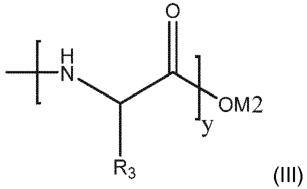
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-f") enthält 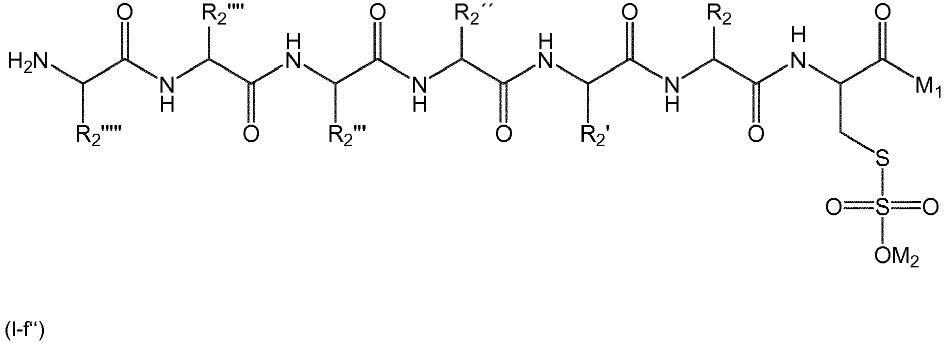 wobei
mindestens einer der Reste aus R2, R2', R2", R2''', R2'''' und R2''''' steht für eine 2-Carboxyethyl-Gruppe (d.h. eine Gruppe HOOC-CH2-CH2-) und
die anderen fünf Reste aus R2, R2', R2", R2''', R2'''' und R2''''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 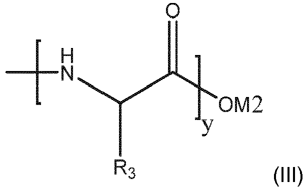
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren ganz besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I) enthält, wobei
R1 für ein Strukturelement der Formel (II) steht, und
x für eine ganze Zahl von mindestens 3 steht und
der Rest R2 in mindestens 3 Strukturelementen der Formel (II) für eine 2-Carboxyethyl-Gruppe steht.

In einer weiteren ganz besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I) enthält, 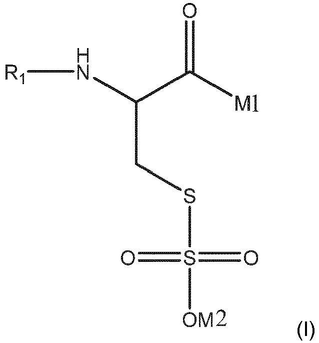 wobei
R1 steht für ein Strukturelement der Formel (II) 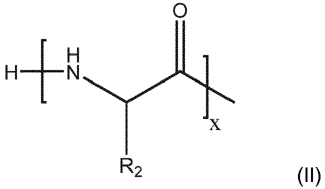 wobei
x für eine ganze Zahl von 3 bis 10 steht,
der Rest R2 in jedem der Strukturelemente der Formel (II) jeweils unabhängig vom vorangegangenen Strukturelement der Formel (II) gewählt werden kann,
der Rest R2 in mindestens 3 Strukturelementen der Formel (II) für eine 2-Carboxyethyl-Gruppe steht,
die anderen Reste R2 stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 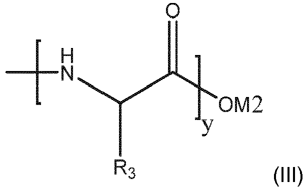
wobei
y für eine ganze Zahl von 1 bis 10 steht
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺.

In einer weiteren ganz besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I) enthält, wobei
M1 für ein Strukturelement der Formel (III) steht, und
der Rest R3 in mindestens einem Strukturelement der Formel (III) für eine (Sulfosulfanyl)methylGruppe (d.h. eine Gruppe HO-S(O₂)-S-CH₂-)steht.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-I') enthält 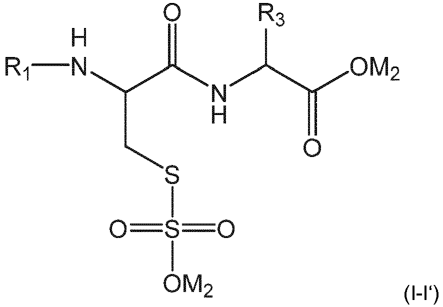 wobei
R1 steht für ein Wasserstoffatom oder für ein Strukturelement der Formel (II) 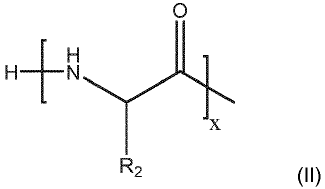 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
R3 steht für eine (Sulfosulfanyl)methyl-Gruppe (d.h. eine Gruppe HO-S(O₂)-S-CH₂-), und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-II') enthält 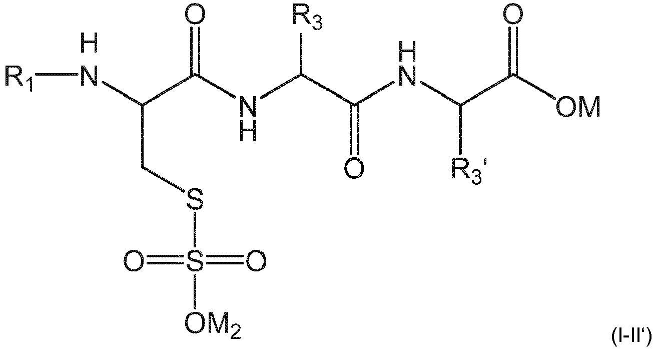 wobei
R1 steht für ein Wasserstoffatom oder für ein Strukturelement der Formel (II) 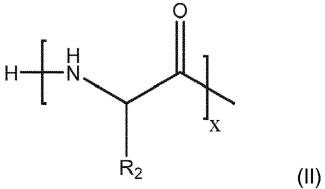 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
einer der Reste aus R3 und R3' steht für eine (Sulfosulfanyl)methyl-Gruppe (d.h. eine Gruppe HO-S(O₂)-S-CH₂-), und
die anderen Reste aus R3 und R3' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-III') enthält 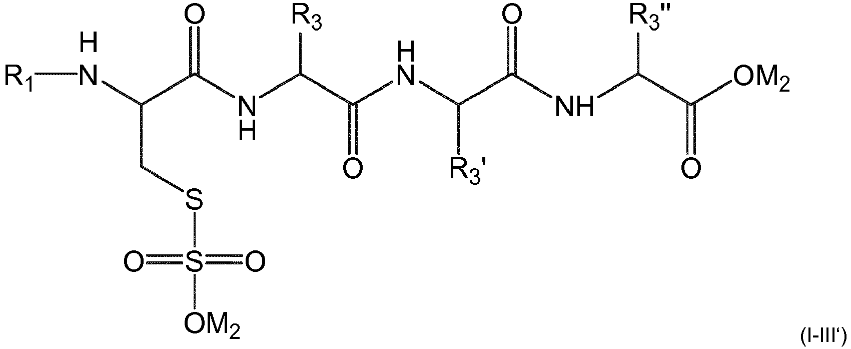 wobei
R1 steht für ein Wasserstoffatom oder für ein Strukturelement der Formel (II) 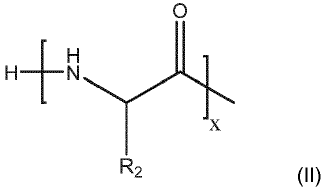 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
einer der Reste aus R3, R3' und R3" steht für eine (Sulfosulfanyl)methyl-Gruppe (d.h. eine Gruppe HO-S(O₂)-S-CH₂-), und
die anderen Reste aus R3, R3' und R3" unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1 H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-IV') enthält 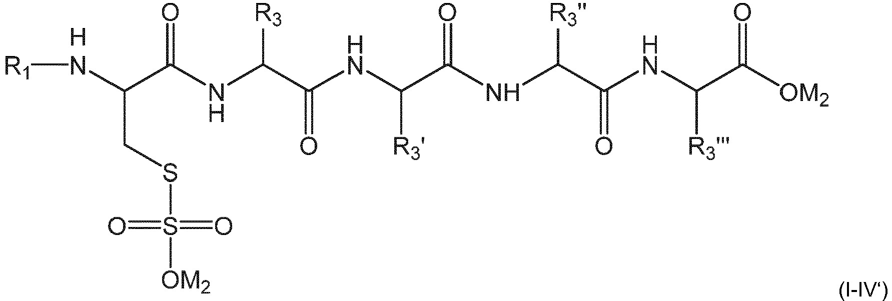 wobei
R1 steht für ein Wasserstoffatom oder für ein Strukturelement der Formel (II) 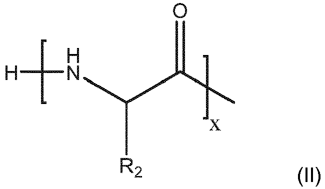 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
einer der Reste aus R3, R3', R3" und R3''' steht für eine (Sulfosulfanyl)methyl-Gruppe (d.h. eine Gruppe HO-S(O₂)-S-CH₂-), und
die anderen Reste aus R3, R3', R3" und R3''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-V') enthält 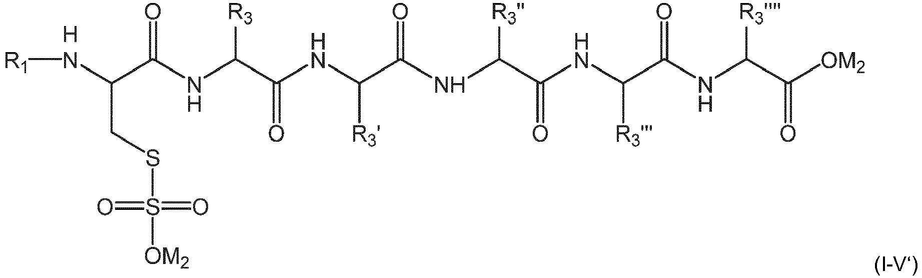 wobei
R1 steht für ein Wasserstoffatom oder für ein Strukturelement der Formel (II) 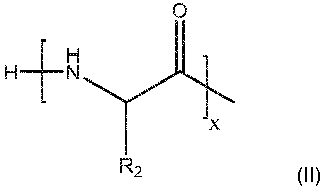 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
einer der Reste aus R3, R3', R3", R3''' und R3'''' steht für eine (Sulfosulfanyl)methyl-Gruppe (d.h. eine Gruppe HO-S(O₂)-S-CH₂-), und
die anderen Reste aus R3, R3', R3", R3''' und R3'''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I-VI') enthält 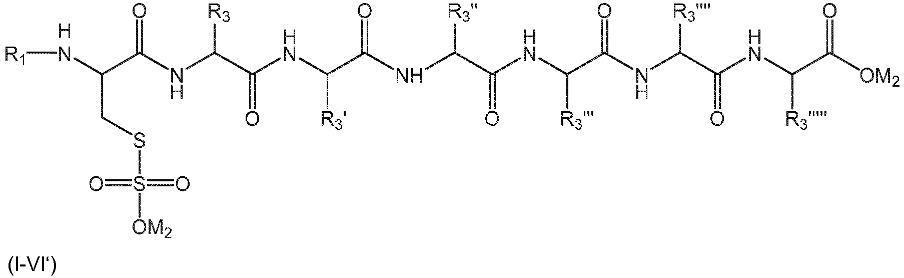 wobei
R1 steht für ein Wasserstoffatom oder für ein Strukturelement der Formel (II) 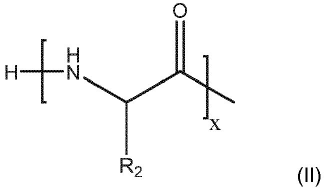 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
einer der Reste aus R3, R3', R3", R3''', R3'''' und R3''''' steht für eine (Sulfosulfanyl)methyl-Gruppe (d.h. eine Gruppe HO-S(O₂)-S-CH₂-), und
die anderen Reste aus R3, R3', R3", R3''', R3'''' und R3''''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I) enthält 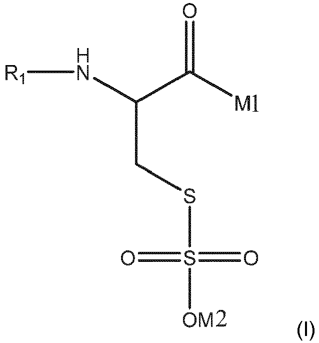 wobei
R1 steht für ein Strukturelement der Formel (II) 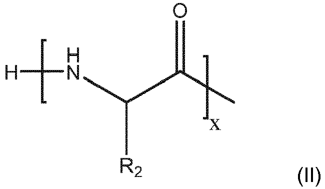 wobei
x für eine ganze Zahl von 3 bis 10 steht
der Rest R2 in jedem der Strukturelemente der Formel (II) jeweils unabhängig vom vorangegangenen Strukturelement der Formel (II) gewählt werden kann,
der Rest R2 in mindestens 3 Strukturelementen der Formel (II) für eine 2-Carboxyethyl-Gruppe (d.h. eine Gruppe HOOC-CH2-CH2-) steht und
der R2 in den verbleidenden Strukturelementen der Formel (II) steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für ein Strukturelement der Formel (III) 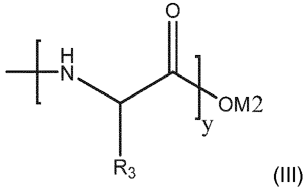
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺.

In einer weiteren ganz besonders bevorzugten Ausführungsform ist eine erfindungsgemäße Mehrkomponenten-Verpackungseinheit zur permanenten Verformung von keratinischen Fasern dadurch gekennzeichnet, dass das Mittel (B) in Container (ii) mindestens eine Verbindung der Formel (I) enthält, wobei
M1 für ein Strukturelement der Formel (III) steht, und
y für eine ganze Zahl von mindestens 1 bis 10 steht und
der Rest R3 in mindestens einem Strukturelement der Formel (III) für eine 2-Carboxyethyl-Gruppe (d.h. eine Gruppe (d.h. eine Gruppe HOOC-CH2-CH2-) steht.

In einer weiteren ganz besonders bevorzugten Ausführungsform ist eine erfindungsgemäße Mehrkomponenten-Verpackungseinheit zur permanenten Verformung von keratinischen Fasern dadurch gekennzeichnet, dass das Mittel (B) in Container (ii) mindestens eine Verbindung der Formel (II") enthält, 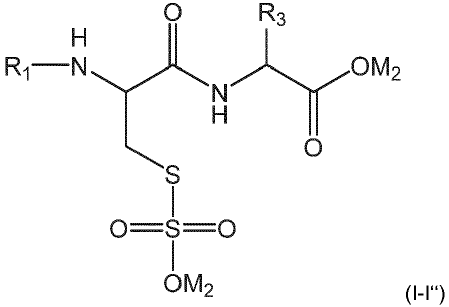 wobei
R1 steht für ein Wasserstoffatom oder für ein Strukturelement der Formel (II) 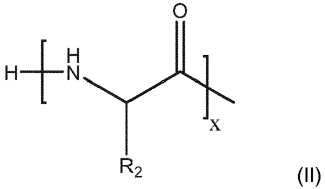 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
R3 steht für eine 2-Carboxyethyl-Gruppe (d.h. eine Gruppe (d.h. eine Gruppe HOOC-CH2-CH2-), und
M2 steht für ein Wasserstoffatom, ein Äquivalent eines ein oder mehrwertigen Kations oder ein Ammoniumion (NH₄)⁺.

In einer weiteren ganz besonders bevorzugten Ausführungsform ist eine erfindungsgemäße Mehrkomponenten-Verpackungseinheit zur permanenten Verformung von keratinischen Fasern dadurch gekennzeichnet, dass das Mittel (B) in Container (ii) mindestens eine Verbindung der Formel (I-II") enthält, 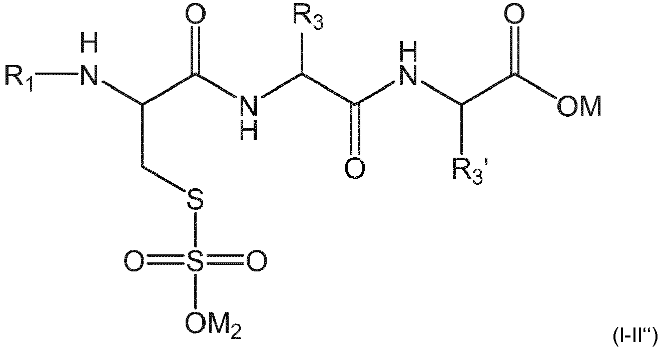 wobei
R1 steht für ein Wasserstoffatom oder für ein Strukturelement der Formel (II) 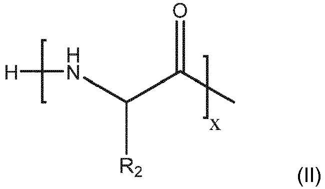 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
einer der Reste aus R3 und R3' steht für eine 2-Carboxyethyl-Gruppe (d.h. eine Gruppe (d.h. eine Gruppe HOOC-CH2-CH2-), und
die anderen Reste aus R3 und R3' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1 H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren ganz besonders bevorzugten Ausführungsform ist eine erfindungsgemäße Mehrkomponenten-Verpackungseinheit zur permanenten Verformung von keratinischen Fasern dadurch gekennzeichnet, dass das Mittel (B) in Container (ii) mindestens eine Verbindung der Formel (I-III') enthält, 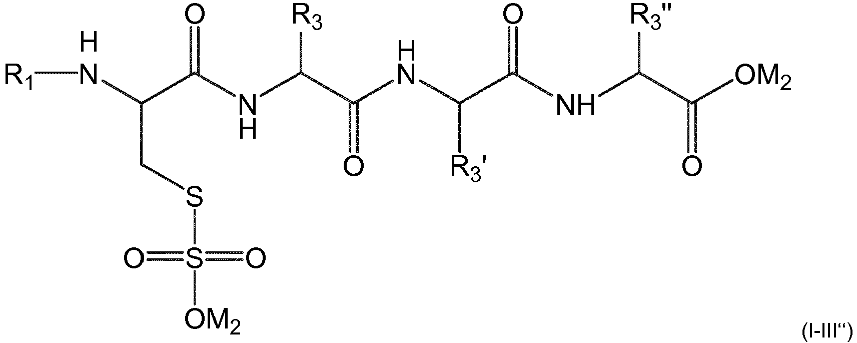 wobei
R1 steht für ein Wasserstoffatom oder für ein Strukturelement der Formel (II) 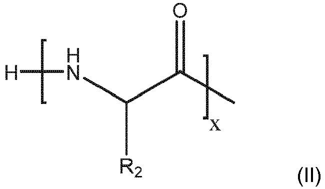 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
einer der Reste aus R3, R3' und R3" steht für eine 2-Carboxyethyl-Gruppe (d.h. eine Gruppe (d.h. eine Gruppe HOOC-CH2-CH2-), und
die anderen Reste aus R3, R3' und R3" unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1 H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren ganz besonders bevorzugten Ausführungsform ist eine erfindungsgemäße Mehrkomponenten-Verpackungseinheit zur permanenten Verformung von keratinischen Fasern dadurch gekennzeichnet, dass das Mittel (B) in Container (ii) mindestens eine Verbindung der Formel (I-IV") enthält, 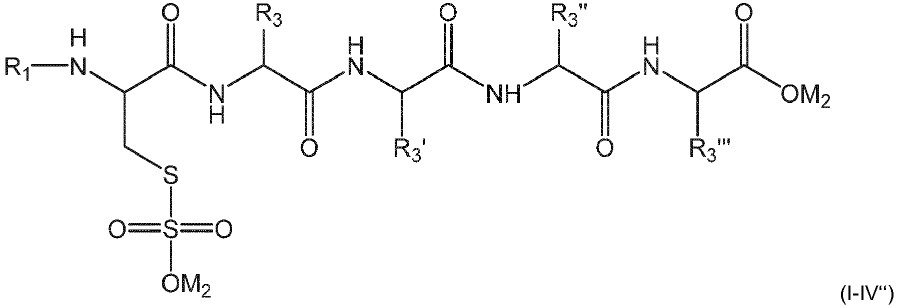 wobei
R1 steht für ein Wasserstoffatom oder für ein Strukturelement der Formel (II) 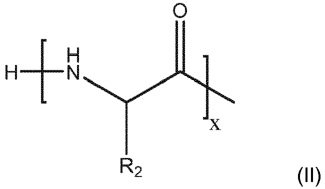 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
einer der Reste aus R3, R3', R3" und R3''' steht für eine 2-Carboxyethyl-Gruppe (d.h. eine Gruppe (d.h. eine Gruppe HOOC-CH2-CH2-), und
die anderen Reste aus R3, R3', R3" und R3''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren ganz besonders bevorzugten Ausführungsform ist eine erfindungsgemäße Mehrkomponenten-Verpackungseinheit zur permanenten Verformung von keratinischen Fasern dadurch gekennzeichnet, dass das Mittel (B) in Container (ii) mindestens eine Verbindung der Formel (I-V") enthält, 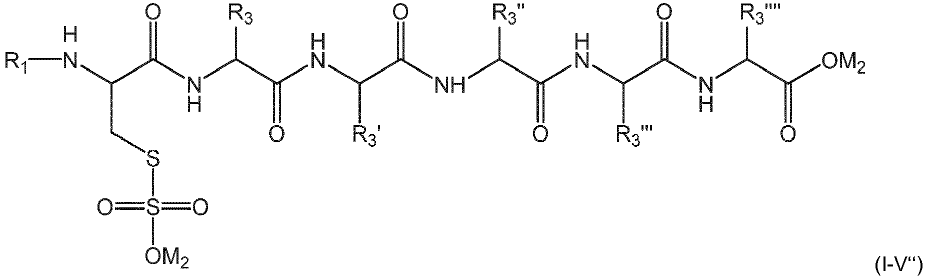 wobei
R1 steht für ein Wasserstoffatom oder für ein Strukturelement der Formel (II) 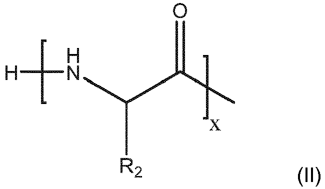 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
einer der Reste aus R3, R3', R3", R3''' und R3'''' steht für eine 2-Carboxyethyl-Gruppe (d.h. eine Gruppe (d.h. eine Gruppe HOOC-CH2-CH2-), und
die anderen Reste aus R3, R3', R3", R3''' und R3'''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺-

In einer weiteren ganz besonders bevorzugten Ausführungsform ist eine erfindungsgemäße Mehrkomponenten-Verpackungseinheit zur permanenten Verformung von keratinischen Fasern dadurch gekennzeichnet, dass das Mittel (B) in Container (ii) mindestens eine Verbindung der Formel (I-VI") enthält, 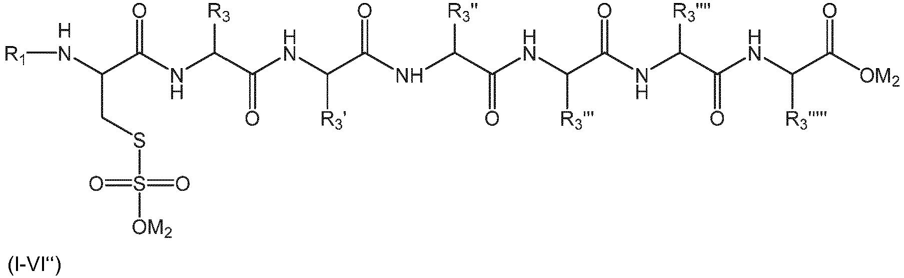 wobei
R1 steht für ein Wasserstoffatom oder für ein Strukturelement der Formel (II) 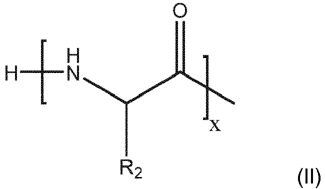 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
einer der Reste aus R3, R3', R3", R3''', R3'''' und R3''''' steht für eine 2-Carboxyethyl-Gruppe (d.h. eine Gruppe (d.h. eine Gruppe HOOC-CH2-CH2-), und
die anderen Reste aus R3, R3', R3", R3''', R3'''' und R3''''' unabhängig voneinander stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺.

In einer weiteren ganz besonders bevorzugten Ausführungsform ist eine erfindungsgemäße Mehrkomponenten-Verpackungseinheit dadurch gekennzeichnet, dass das Mittel (B) in Container (ii) mindestens eine Verbindung der Formel (I) enthält, wobei
M1 für ein Strukturelement der Formel (III) steht, und
y für eine ganze Zahl von mindestens 3 steht und
der Rest R3 in mindestens 3 Strukturelementen der Formel (III) für eine 2-Carboxyethyl-Gruppe (d.h. eine Gruppe HOOC-CH2-CH2-) steht.

In einer weiteren ganz besonders bevorzugten Ausführungsform ist eine erfindungsgemäße Mehrkomponenten-Verpackungseinheit zur permanenten Verformung von keratinischen Fasern dadurch gekennzeichnet, dass das Mittel (B) in Container (ii) mindestens eine Verbindung der Formel (I) enthält, 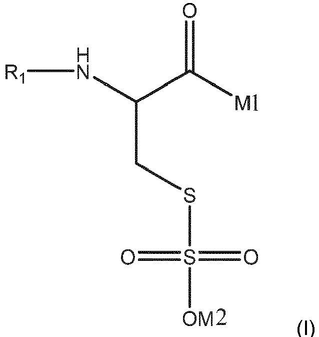 wobei
R1 steht für ein Strukturelement der Formel (II) 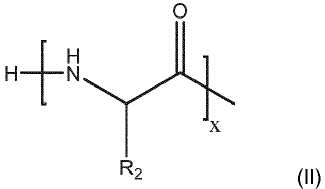 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für ein Strukturelement der Formel (III) 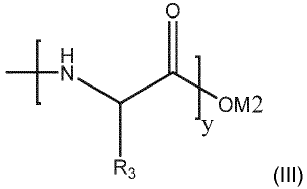
wobei
y für eine ganze Zahl von 3 bis 10 steht,
der Rest R3 in jedem der Strukturelemente der Formel (III) jeweils unabhängig vom vorangegangenen Strukturelement der Formel (III) gewählt werden kann,
der Rest R3 in mindestens drei Strukturelementen der Formel (III) für eine 2-Carboxyethyl-Gruppe (d.h. eine Gruppe (d.h. eine Gruppe HOOC-CH2-CH2-) steht, und
der Rest R3 in den verbleibenden Strukturelementen der Formel (III) steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺.

In einer weiteren ganz besonders bevorzugten Ausführungsform ist eine erfindungsgemäße Mehrkomponenten-Verpackungseinheit zur permanenten Verformung von keratinischen Fasern dadurch gekennzeichnet, dass das Mittel (B) in Container (ii) mindestens eine Verbindung der Formel (I) enthält, 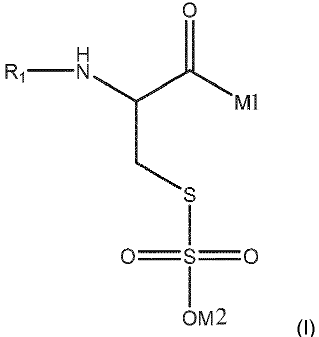 wobei
R1 steht für ein Strukturelement der Formel (II) 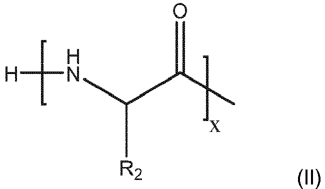 wobei
x für eine ganze Zahl von 8 bis 10 steht
der Rest R2 in jedem der Strukturelemente der Formel (II) jeweils unabhängig vom vorangegangenen Strukturelement der Formel (II) gewählt werden kann,
   - einer der Reste R2 für eine 2-Methylpropylgruppe steht und
   - einer der Reste R2 für eine Isopropylgruppe steht und
   - einer der Reste R2 für eine 3-Carbamimidamidopropyl-Gruppe (d.h. eine Gruppe H₂N-C(NH)-NH-CH₂-CH₂-CH₂-) steht und
   - einer der Reste R2 für eine 1-Methyl-propylgruppe (d.h. eine Gruppe H3C-CH2-CH(CH3)-) steht und
   - drei der Reste R2 für eine 2-Carboxyethyl-Gruppe (d.h. eine Gruppe (d.h. eine Gruppe HOOC-CH2-CH2-) stehen und
   - einer der Reste Rest R2 für eine 4-Hydroxybenzylgruppe (d.h. eine Gruppe 4-OH-C₆H₅-CH₂-) steht
   - die weiteren Reste R2 stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 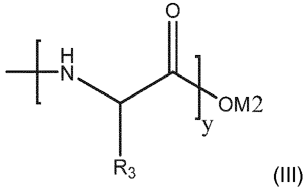
wobei
y für eine ganze Zahl von 1 bis 10 steht,
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺ und
(b) mindestens eine Keratin reduzierende Verbindung, die mindestens eine Thiolgruppe (-SH) und/oder mindestens eine Disulfidgruppe (-S-S-) besitzt

In einer weiteren ganz besonders bevorzugten Ausführungsform ist eine erfindungsgemäße Mehrkomponenten-Verpackungseinheit zur permanenten Verformung von keratinischen Fasern dadurch gekennzeichnet, dass das Mittel (B) in Container (ii) mindestens eine Verbindung der Formel (I) enthält, 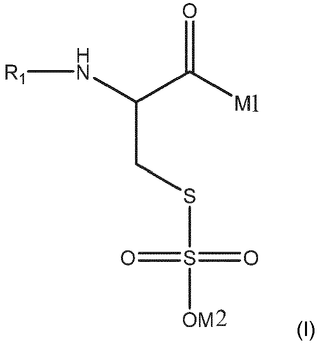 wobei
R1 steht für ein Wasserstoffatom oder für ein Strukturelement der Formel (II) 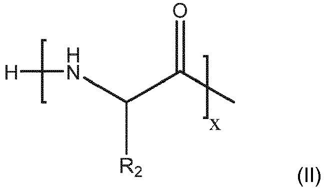 wobei
x für eine ganze Zahl von 1 bis 10 steht
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für ein Strukturelement der Formel (III) 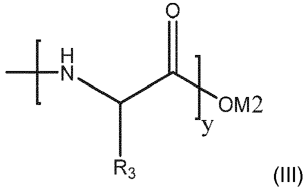
wobei
y für eine ganze Zahl von 8 bis 10 steht,
der Rest R3 in jedem der Strukturelemente der Formel (III) jeweils unabhängig vom vorangegangenen Strukturelement der Formel (III) gewählt werden kann,
   - einer der Reste R3 für eine 2-Methylpropylgruppe steht und
   - einer der Reste R3 für eine Isopropylgruppe steht und
   - einer der Reste R3 für eine 3-Carbamimidamidopropyl-Gruppe (d.h. eine Gruppe H₂N-C(NH)-NH-CH₂-CH₂-CH₂-) steht und
   - einer der Reste R3 für eine 1-Methyl-propylgruppe (d.h. eine Gruppe H3C-CH2-CH(CH3)-) steht und
   - drei der Reste R3 für eine 2-Carboxyethyl-Gruppe (d.h. eine Gruppe (d.h. eine Gruppe HOOC-CH2-CH2-) stehen und
   - einer der Reste Rest R3 für eine 4-Hydroxybenzylgruppe (d.h. eine Gruppe 4-OH-C₆H₅-CH₂-) steht
   - die weiteren Reste R3 stehen für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe, und
M2 steht für ein Wasserstoffatom, ein Äquivalent eines ein oder mehrwertigen Kations oder ein Ammoniumion (NH₄)⁺-

Die Verbindungen der Formel (I) können - bezogen auf das Gesamtgewicht des Mittels (B) - in einer Gesamtmenge von 0,0001 bis 10 Gew.-% eingesetzt werden. Überraschenderweise hat sich jedoch herausgestellt, dass die Verbindung(en) der Formel (I) bereits in geringen Einsatzkonzentrationen eine sehr gute Reduzierung der Haarschädigung und Verbesserung der Wellwirkung bewirken können. Aus diesem Grund ist es besonders vorteilhaft, wenn das Mittel (B) - bezogen auf sein Gesamtgewicht eine oder mehrere Verbindungen der Formel (I) in einer Gesamtmenge von 0,001 bis 10,0 Gew.-%, bevorzugt von 0,01 bis 2,5 Gew.-% weiter bevorzugt von 0,05 bis 1,0 Gew.-% und besonders bevorzugt von 0,05 bis 0,3 Gew.-% enthält.

In einer weiteren ganz besonders bevorzugten Ausführungsform ist eine erfindungsgemäß Mehrkomponenten-Verpackungseinheit dadurch gekennzeichnet, dass das Mittel (B) in Container (ii) - bezogen auf das Gesamtgewicht des Mittels (B) - eine oder mehrere Verbindungen der Formel (I) in einer Gesamtenge von 0,0001 bis 10,0 Gew.-%, bevorzugt 0,001 bis 10,0 Gew.-%, weiter bevorzugt von 0,01 bis 2,5 Gew.-%, noch weiter bevorzugt von 0,05 bis 1,0 Gew.-% und besonders bevorzugt von 0,05 bis 0,3 Gew.-% enthält.

### Mehrkomponenten-Verpackungseinheit (Kit-of-Parts), weitere Komponente(n)

Bei dem zuvor beschriebenen Mittel (A) handelt es sich um ein Mittel zur permanenten Verformung der Haare, das zur Reduktion der im Haar befindlichen Cystin-Disulfid-Bindungen auf die Haare aufgetragen wird. Üblicherweise werden die Haare vor, während oder nach der Auftragung der Reduktionsmittelformulierung mechanisch verformt.

Das Mittel (B) enthält das oder die zuvor beschriebenen Bunte-Salze der Formel (I). Das Mittel (B) kann beispielsweise vor, während oder nach der Anwendung des Mittels (A) appliziert werden. In diesem Zusammenhang sind verschiedene Anwendungsprozesse möglich und bevorzugt:
Die Haare werden mechanisch verformt. Dann wird das Mittel (A) auf die Haare appliziert und anschließend wieder ausgespült. Im Anschluss daran wird das Mittel (B) appliziert.

Die Haare werden mechanisch verformt. Dann wird das Mittel (A) auf die Haare appliziert. Im Anschluss daran wird das Mittel (B) appliziert, jedoch ohne dass das Mittel (A) vorher ausgewaschen worden wäre.

Wenn die Haare zur mechanischen Verformung auf Wickler gedreht wurden, konnte durch zuvor beschriebenen Applikationsmethoden eine Verstärkung der Wellwirkung erzielt werden. Darüber besaßen die Locken eine Längere Haltbarkeit, d.h. die Locken hingen sich nicht so schnell aus.

Um die zuvor verformten Haarsträhnen nun in ihrer neuen Form zu fixieren, wird nach dem Auswaschen des Reduktionsmittels (und/oder des Mittels (B)) nun eine Fixierlösung auf die Haare aufgetragen.

Die Fixierlösung enthält ein Oxidationsmittel (meist Wasserstoffperoxid) und gewährleistet die Rückoxidation der bis zu diesem Zeitpunkt noch in Thiolform vorliegenden Cystein-Anteile des Haares.

Prinzipiell kann das Oxidationsmittel auch dem Mittel (B) zugesetzt werden, so dass das Mittel (B) gleichzeitig das bzw. die Bunte-Salze der Formel (I) und das Oxidationsmittel (insbesondere Wasserstoffperoxid) enthält. Als bevorzugt hat sich jedoch herausgestellt, der Mehrkomponenten-Verpackungseinheit die Fixierlösung in Form einer weiteren dritten, getrennt konfektionierten Zubereitung hinzuzufügen. Im Rahmen dieser weiteren ganz besonders bevorzugten Ausführungsform umfasst die erfindungsgemäße Mehrkomponenten-Verpackungseinheit daher neben den Containern (i) und (ii), welche die Mittel (A) und (B) enthalten, noch einen dritten Container (iii), in dem sich das Fixiermittel (C) befindet.

Weiterhin ganz besonders bevorzugt ist eine erfindungsgemäße Mehrkomponenten-Verpackungseinheit, umfassend getrennt konfektioniert
- einen Container (iii) enthaltend ein kosmetisches Mittel (C), wobei
- das Mittel (C) mindestens ein Oxidationsmittel enthält.

Das Fixiermittel (d.h. das kosmetische Mittel (C)) enthält das Oxidationsmittel, insbesondere Wasserstoffperoxid, in einem kosmetischen Träger. In einer bevorzugten Ausführungsform wird Wasserstoffperoxid selbst als wässrige Lösung im Fixiermittel (C) verwendet. Die Konzentration einer Wasserstoffperoxid-Lösung wird hierbei einerseits von den gesetzlichen Vorgaben und andererseits von dem gewünschten Effekt bestimmt; vorzugsweise werden 1,5 bis 12 Gew.-%ige Lösungen in Wasser verwendet. Erfindungsgemäß bevorzugte Fixiermittel (c) sind dadurch gekennzeichnet, dass sie 1,0 bis 15,0 Gew.-%, bevorzugt 2,0 bis 12,0 Gew.-%, weiter bevorzugt 3,0 bis 9,0 Gew.-% und ganz besonders bevorzugt 3,5 bis 7,5 Gew.-% Wasserstoffperoxid, jeweils bezogen auf das Gesamtgewicht des Fixiermittels (c), enthalten.

In einer weiteren besonders bevorzugten Ausführungsform ist eine erfindungsgemäße Mehrkomponenten-Verpackungseinheit dadurch gekennzeichnet, dass das Mittel (C) in Container (iii) als Oxidationsmittel Wasserstoffperoxid enthält.

In einer weiteren besonders bevorzugten Ausführungsform ist eine erfindungsgemäße Mehrkomponenten-Verpackungseinheit dadurch gekennzeichnet, dass das Mittel (C) in Container (iii) - bezogen auf das Gesamtgewicht des Mittels (C) - 1,0 bis 15,0 Gew.-%, bevorzugt 2,0 bis 12,0 Gew.-%, weiter bevorzugt 3,0 bis 9,0 Gew.-% und ganz besonders bevorzugt 3,5 bis 7,5 Gew.-% Wasserstoffperoxid enthält.

Neben den Containern (i) und (ii) (sowie ggf. Container (iii)) kann die erfindungsgemäße Mehrkomponenten-Verpackungseinheit auch noch mindestens einen weiteren Container umfassen. Dieser vierte Container (iv) kann beispielsweise das Mittel (D) enthalten, bei dem es sich ein weiteres Vorbehandlungsmittel und/oder Nachbehandlungsmittel handeln kann. Das Mittel (D) kann ein Shampoo, ein Conditioner, oder aber auch ein Mittel vom Typ eines Pflegetropfens sein, wobei der Pflegetropfen beispielsweise vor der Anwendung mit dem Mittel (A) und/oder (B) (und/oder (C)) vermischt werden kann.

Bei diesem vierten Mittel (D) handelt es sich ganz besonders bevorzugt um eine wässrige, sauer eingestellte Pflege-Formulierung.

In einer weiteren Ausführungsform besonders bevorzugt ist daher eine Mehrkomponenten-Verpackungseinheit, umfassend getrennt konfektioniert
- einen Container (iv) enthaltend ein kosmetisches Mittel (D),
   wobei
- das Mittel (D) in einem wässrigen kosmetischen Träger mindestens eine Säure enthält und einen pH-Wert von 1,5 bis 5,5, bevorzugt von 2,0 bis 5,0, weiter bevorzugt von 2,5 bis 4,5 und ganz besonders bevorzugt von 2,5 bis 4,0 besitzt.

Als bevorzugt hat es sich herausgestellt, das Mittel (A) auf einen alkalischen pH-Wert einzustellen, der vorteilhafter Weise im Bereich von 7,0 bis 10,0 liegt. Zur Einstellung des alkalischen pH-Wertes können die aus der Kosmetik bekannten und für die Kosmetik zugelassenen Alkalisierungsmittel eingesetzt werden. Besonders geeignet sind beispielsweise Ammoniak, C1-C4-Alkanolamine oder anorganische Hydroxide.

Die Mittel (B), (C) und/oder (D) werden bevorzugt auf einen neutral bis sauren pH-Wert eingestellt. Die Einstellung des sauren pH-Wertes kann prinzipiell mit verschiedenen Säuren erfolgen. Geeignete Säuren sind beispielsweise Zitronensäure, Weinsäure, Äpfelsäure, Milchsäure, Essigsäure, Schwefelsäure, Salzsäure, Phosphorsäure, Methansulfonsäure, Benzoesäure Malonsäure, Oxalsäure und/oder 1-Hydroxyethan-1,1-diphosphonsäure als geeignet erwiesen. Bevorzugt wird bzw. werden die Säuren aus der Gruppe aus Zitronensäure, Weinsäure, Äpfelsäure, Milchsäure, Methansulfonsäure, Oxalsäure, Malonsäure, Benzoesäure, Salzsäure, Schwefelsäure, Phosphorsäure und/oder 1-Hydroxyethan-1,1-diphosphonsäure.

In einer weiteren ganz besonders bevorzugten Ausführungsform ist eine erfindungsgemäße Mehrkomponenten-Verpackungseinheit dadurch gekennzeichnet, dass
- das Mittel (A) in Container (i) Wasser enthält und einen pH-Wert von 7,0 bis 10,0, bevorzugt von 7,5 bis 9,5 besitzt,
- das Mittel (B) in Container (ii) Wasser enthält und einen pH-Wert von 2,5 bis 6,5 bevorzugt von 3,0 bis 5,5 besitzt und
- das Mittel (C) in Container (iii) Wasser enthält und einen pH-Wert von 1,5 bis 5,5, bevorzugt von 2,5 bis 4,5 besitzt.

Alle zuvor beschriebenen Mittel - d.h. das Mittel (A) und (B), sowie auch die weiteren optional vorhandenen Mittel (C) und (D) - können auch noch weitere Inhaltsstoffe enthalten. So können die Mittel zusätzlich auch organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel, wobei das Lösungsmittel in einer Gesamtmenge von 0,1 bis 30 Gew.-%, vorzugsweise von 1 bis 20 Gew.-%, insbesondere von 2 bis 10 Gew.-%, bezogen auf das Gesamtgewicht des jeweiligen kosmetischen Mittels, enthalten ist.

Die Mittel - d.h. das Mittel (A) und (B), sowie auch die weiteren optional vorhandenen Mittel (C) und (D) - können zusätzlich mindestens einen weiteren konditionierenden Stoff, ausgewählt aus der Gruppe von kationischen Polymeren, katonischen Tensiden, Siliconen, Fettstoffen sowie deren Mischungen, enthalten. Unter dem Begriff "konditionierende Stoffe" sind erfindungsgemäß solche Substanzen zu verstehen, welche auf keratinische Materialien, insbesondere auf die Haare, aufziehen und die physikalischen und sensorischen Eigenschaften sowohl der Haare als auch des Produktes als solchem verbessern. Konditionierungsmittel glätten die oberste Schicht der Haare und machen sie weich und geschmeidig.

Bevorzugte kationische Polymere sind ausgewählt aus der Gruppe von Poly(methacryloyloxyethyl-trimethylammoniumchlorid) (INCI: Polyquaternium-37), quaternisierten Cellulose-Derivaten (INCI: Polyquaternium 10), kationischen Alkylpolyglycosiden, kationisiertem Honig, kationischen Guar-Derivaten, polymeren Dimethyldiallylammoniumsalzen und deren Copolymeren mit Estern und Amiden von Acrylsäure und Methacrylsäure, Copolymeren des Vinylpyrrolidons mit quaternierten Derivaten des Dialkylaminoalkylacrylats und -methacrylats, Vinylpyrrolidon-Vinylimidazoliummethochlorid-Copolymeren, quaterniertem Polyvinylalkohol, Polyquaternium 2, Polyquaternium-7, Polyquaternium 17, Polyquaternium 18, Polyquaternium 24, Polyquaternium 27 sowie deren Mischungen.

Im Rahmen der vorliegenden Erfindung kann es bevorzugt sein, wenn das/die kationische(n) Polymer(e) in einer Gesamtmenge von 0,05 bis 7,5 Gew.-%, vorzugsweise von 0,1 bis 5 Gew.-%, bevorzugt von 0,2 bis 3,5 Gew.-%, insbesondere von 0,25 bis 2,5 Gew.-%, bezogen auf das Gesamtgewicht des jeweiligen kosmetischen Mittels, enthalten sind.

Als konditionierende Stoffe eignen sich im Rahmen der vorliegenden Erfindung ebenfalls kationische Tenside aus der Gruppe der quartären Ammoniumverbindungen und/oder der Esterquats und/oder der Amidoamine.

Bevorzugt verwendete kationische Tenside sind ausgewählt aus der Gruppe von Alkyltrimethylammoniumchloriden mit vorzugsweise 10 bis 18 Kohlenstoffatomen im Alkylrest, Dialkyldimethylammoniumchloride mit vorzugsweise 10 bis 18 Kohlenstoffatomen im Alkylrest, Trialkylmethylammoniumchloride mit vorzugsweise 10 bis 18 Kohlenstoffatomen im Alkylrest, Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid, Tricetylmethylammoniumchlorid, Quaternium-27, Quaternium-83, N-Methyl-N(2-hydroxyethyl)-N,N-(ditalgacyloxyethyl)ammonium-methosulfat, N-Methyl-N(2-hydroxyethyl)-N,N-(distearoyloxyethyl)ammonium-methosulfat, N,N-Dimethyl-N,N-distearoyloxyethyl-ammoniumchlorid, N,N-Di-(2-hydroxyethyl)-N,N-(fettsäureesterethyl)-ammoniumchlorid sowie deren Mischungen.

Das/die kationische(n) Tensid(e) sind in den erfindungsgemäß verwendeten kosmetischen Mitteln bevorzugt in einer Gesamtmenge von 0,5 bis 50 Gew.-%, vorzugsweise von 1 bis 40 Gew.-%, besonders bevorzugt von 1,5 bis 30 Gew.-%, insbesondere von 2 bis 20 Gew.-%, bezogen auf das Gesamtgewicht des jeweiligen kosmetischen Mittels, enthalten.

Erfindungsgemäß geeignete Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül.

Erfindungsgemäß geeignete Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß geeignete Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf das Gesamtgewicht des jeweiligen Mittels, eingesetzt.

Die erfindungsgemäßen Mittel können auch mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere, kationische, synthetische Polymere, natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen, nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Weiterhin hat es sich als vorteilhaft erweisen, wenn die erfindungsgemäßen Mittel insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die jeweiligen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, jeweils bezogen auf das Gesamtgewicht des jeweiligen Mittels.

### Verfahren zur permanenten Formveränderung von keratinischen Fasern

Die Mittel, die mit der erfindungsgemäßen Mehrkomponenten-Verpackungseinheit des ersten Erfindungsgegenstands zur Verfügung gestellt werden, werden in Verfahren zur permanenten Formveränderung von keratinischen Fasern, insbesondere menschlichen Haaren, eingesetzt.

Ein dritter Gegenstand der vorliegenden Erfindung ist deshalb ein Verfahren zur permanenten Formveränderung von keratinischen Fasern, insbesondere menschlichen Haaren, umfassend die folgenden Schritte
a1) Applizieren eines kosmetischen Mittels (A) auf die keratinischen Fasern,
a2) Einwirkenlassen des Mittels (A) für einen Zeitraum von 2 bis 60 Minuten,
a3) gegebenenfalls Ausspülen des Mittels (A) aus den keratinischen Fasern,
b1) Applizieren eines kosmetischen Mittels (B) auf die keratinischen Fasern,
b2) Einwirkenlassen des Mittels (B) für einen Zeitraum von 2 bis 60 Minuten,
b3) gegebenenfalls Ausspülen des kosmetischen Mittels (B) aus den keratinischen Fasern,
c1) Applizieren eines kosmetischen Mittels (C) auf die keratinischen Fasern,
c2) Einwirkenlassen des Mittels (C) für einen Zeitraum von 2 bis 60 Minuten,
c3) Ausspülen des Mittels (C) aus den keratinischen Fasern,
   wobei
   - die Mittel (A), (B) und (C) die Mittel sind, wie sie bei der Beschreibung des ersten Erfindungsgegenstands im Detail offenbart wurden.

Die Mittel (A), (B) und (C) sind die Mittel wie sie bei der Beschreibung des ersten Erfindungsgegenstands im Detail offenbart wurden, d.h. das Mittel (A) wird aus einem ersten Container (i) appliziert und enthält mindestens eine Keratin reduzierende Verbindung. Das Mittel (B) wird aus einem zweiten Container (ii) appliziert und enthält mindestens eine Verbindung der allgemeinen Formel (I). Das Mittel (C) wird schließlich aus einem dritten Container (iii) appliziert, stellt das Fixiermittel dar und enthält mindestens ein Oxidationsmittel, insbesondere Wasserstoffperoxid

Innerhalb dieses Verfahrens sind verschiedene Schrittabfolgen besonders geeignet.

Ganz besonders bevorzugt ist ein Verfahren, umfassend die folgenden Schritte in der angegebenen Reihenfolge:
a1) Applizieren eines kosmetischen Mittels (A) auf die keratinischen Fasern,
a2) Einwirkenlassen des Mittels (A) für einen Zeitraum von 2 bis 60 Minuten,
a3) Ausspülen des Mittels (A) aus den keratinischen Fasern,
b1) Applizieren eines kosmetischen Mittels (B) auf die keratinischen Fasern,
b2) Einwirkenlassen des Mittels (B) für einen Zeitraum von 2 bis 60 Minuten,
b3) gegebenenfalls Ausspülen des kosmetischen Mittels (B) aus den keratinischen Fasern,
c1) Applizieren eines kosmetischen Mittels (C) auf die keratinischen Fasern,
c2) Einwirkenlassen des Mittels (C) für einen Zeitraum von 2 bis 60 Minuten,
c3) Ausspülen des Mittels (C) aus den keratinischen Fasern,

Im Rahmen dieser Ausführungsform werden beispielsweise zunächst die Haare auf Wickler gedreht, dann wird das Mittel (A) angewendet, einwirken gelassen und wieder ausgespült. Im Rahmen dieser Ausführungsform dient das Mittel (B) als Zwischenbehandlungsmittel und wird nach dem Reduktionsmittel, aber vor dem Fixiermittel (C) auf die Haare appliziert. Das Mittel (B) kann ausgespült werden. Es ist aber ebenfalls möglich, das Mittel (B) nicht auszuspülen, sondern das Fixiermittel (C) auf die Haare aufzutragen, die noch mit dem Mittel (B) beaufschlagt sind. In diesem Fall werden dann die Mittel (B) und (C) zusammen in Schritt c3) ausgespült. Bei Durchführung dieses Verfahrens kann eine weitere Verstärkung der Wellwirkung gewährleistet werden. Weiterhin hat sich herausgestellt, dass die Schädigung der Haare auf diesem Wege reduziert werden konnte.

Bevorzugt ist auch ein Verfahren, umfassend die folgenden Schritte in der angegebenen Reihenfolge:
a1) Applizieren eines kosmetischen Mittels (A) auf die keratinischen Fasern,
a2) Einwirkenlassen des Mittels (A) für einen Zeitraum von 2 bis 60 Minuten,
b1) Applizieren eines kosmetischen Mittels (B) auf die keratinischen Fasern,
b2) Einwirkenlassen des Mittels (B) für einen Zeitraum von 2 bis 60 Minuten,
b3) Ausspülen der kosmetischen Mittel (A) und (B) aus den keratinischen Fasern,
c1) Applizieren eines kosmetischen Mittels (C) auf die keratinischen Fasern,
c2) Einwirkenlassen des Mittels (C) für einen Zeitraum von 2 bis 60 Minuten,
c3) Ausspülen des Mittels (C) aus den keratinischen Fasern,

Im Rahmen dieser Ausrührungsform werden beispielsweise zunächst die Haare auf Wickler gedreht, dann wird das Mittel (A) angewendet und einwirken gelassen. Dass Mittel (A) wird jedoch nicht ausgespült, sondern das Mittel (B) wird auf die Haare - die noch mit dem Mittel (A) beaufschlagt sind - aufgetragen. In Schritt b3) werden dann das Mittel (A) und das Mittel (B) zusammen aus den Haaren ausgespült. Nach dem Ausspülen der Mittel (A)/(B) wird dann wieder das Fixiermittel (C) auf die Haare appliziert, einwirken gelassen und wieder ausgespült.

Besonders bevorzugt ist weiterhin ein Verfahren, umfassend die folgenden Schritte in der angegebenen Reihenfolge:
a1) Applizieren eines kosmetischen Mittels (A) auf die keratinischen Fasern,
a2) Einwirkenlassen des Mittels (A) für einen Zeitraum von 2 bis 60 Minuten,
a3) Ausspülen des Mittels (A) aus den keratinischen Fasern,
c1) Applizieren eines kosmetischen Mittels (C) auf die keratinischen Fasern,
c2) Einwirkenlassen des Mittels (C) für einen Zeitraum von 2 bis 60 Minuten,
c3) Ausspülen des Mittels (C) aus den keratinischen Fasern,
b1) Applizieren eines kosmetischen Mittels (B) auf die keratinischen Fasern,
b2) Einwirkenlassen des Mittels (B) für einen Zeitraum von 2 bis 60 Minuten,
b3) gegebenenfalls Ausspülen des kosmetischen Mittels (B) aus den keratinischen Fasern.

Im Rahmen dieser Ausführungsform werden die Keratinfasern, bevorzugt Haare, beispielsweise auf Wickler gedreht und konventionell durch sukzessive Anwendung der Mittel (A) (Dauerwellmittel) und (C) (Fixiermittel) verformt. Danach wird dann das Mittel (B) als Nachbehandlungsmittel auf die Fasern (Haare) aufgetragen. In diesem Zusammenhang kann das Mittel (B) sowohl als "leave-on" als auch als "rinse-off" Mittel konfektioniert werden. Wird das Mittel (B) als "leave-on" Mittel konfektioniert, verbleibt es auf der Anwendung auf den Keratinfasern (dem Haar). Wird das Mittel (B) als "rinse-off" Mittel konfektioniert, wird es nach Anwendung und Einwirken wieder ausgespült.

Ebenfalls erfindungsgemäß und geeignet ist auch ein Verfahren, umfassend die folgenden Schritte in der angegebenen Reihenfolge:
a1) Applizieren eines kosmetischen Mittels (A) auf die keratinischen Fasern,
a2) Einwirkenlassen des Mittels (A) für einen Zeitraum von 2 bis 60 Minuten,
a3) Ausspülen des Mittels (A) aus den keratinischen Fasern,
c1) Applizieren eines kosmetischen Mittels (C) auf die keratinischen Fasern,
c2) Einwirkenlassen des Mittels (C) für einen Zeitraum von 2 bis 60 Minuten,
b1) Applizieren eines kosmetischen Mittels (B) auf die keratinischen Fasern,
b2) Einwirkenlassen des Mittels (B) für einen Zeitraum von 2 bis 60 Minuten,
b3) Ausspülen der kosmetischen Mittel (B) und (C) aus den keratinischen Fasern.

Im Rahmen dieser Ausführungsform werden die Keratinfasern, bevorzugt Haare, beispielsweise auf Wickler gedreht und konventionell durch sukzessive Anwendung der Mittel (A) (Dauerwellmittel) und (C) (Fixiermittel) verformt. Vor der Anwendung des Mittels (B) wird das Mittel (C) jedoch nicht ausgespült. In diesem Fall erfolgt in Schritt b3) ein gemeinsames Ausspülen der Mittel (B) und (C).

Die zuvor beschriebenen Mehrkomponenten-Verpackungseinheiten und Verfahren zur permanenten Verformen können die Keratinfasern sowohl glätten als auch dauerwellen.

Zum Dauerwellen können die Haare bevorzugt vor dem Applizieren des Mittels (A) auf Papilloten oder Lockenwickler aufgedreht. Zum Glätten werden die Haare bevorzugt während des Einwirkens des Mittels (A) oder nach dessen Ausspülen mit einem Kamm oder mit einer Bürste glatt gekämmt.

In einer besonders bevorzugten Ausführungsform ist das erfindungsgemäße Verfahren deshalb dadurch gekennzeichnet, dass die keratinischen Fasern unter Zuhilfenahme von Verformungshilfsmitteln vor, nach oder während dem Schritt a1), a2) oder a3) verformt werden.

Verformungshilfsmittel sind beispielsweise Wickler, Papilloten, Kämme, Bürsten.

Bezüglich der besonders bevorzugten Ausführungsformen erfindungsgemäßen Verfahrens gilt mutatis mutandis das zu der erfindungsgemäßen Mehrkomponenten-Verpackungseinheit gesagte.

Die Haarschädigung mittels verschiedener physikalischer Messmethoden festgestellt bzw. quantifiziert werden. Beispielsweise kann der Cystein-Wert der Haare über IR-Messungen bestimmt werden, das Zug-Dehnungsverhalten der Haare kann gemessen werden, oder aber die Haar können im Hinblick auf ihre Kämmbarkeit oder das Ausmaß des auftretenden Spliss- und Haarbruchs beurteilt werden.

Bezüglich der besonders bevorzugten Ausführungsformen der Verwendung der Verbindungen der Formel (I) gilt mutatis mutandis das zu dem erfindungsgemäßen Mehrkomponenten-Verpackungseinheiten und Verfahren gesagte.

Beispiele

### 1. Formulierungen

Es wurden folgende Zusammensetzungen hergestellt (alle Mengenangaben in Gew.-%)

### Mittel (A)

| | A1 | A2 | A3 |
|---|---|---|---|
| Ammoniumthioglykolat | 11,8 | 9,5 | 10,2 |
| Turpinal SL | 0,30 | 0,30 | 0,30 |
| Ammoniumcarbonat | 2,50 | 2,50 | 2,50 |
| Cremophor CO 40 | 0,75 | 0,75 | 0,75 |
| Protelan VE/K | 0,80 | 0,80 | 0,80 |
| Ammoniak (25%ige wässrige Lösung) | ad pH 8,3 | ad pH 8,3 | ad pH 8,3 |
| Parfum | 0,50 | 0,50 | 0,50 |
| Wasser | ad 100 | ad 100 | ad 100 |

| | |
|---|---|
| Cremophor® CO40 | hydriertes Rizinusöl mit ca. 40-45 EO-Einheiten (INCI-Bezeichnung: PEG-40 Hydrogenated Castor Oil) (BASF) |
| Protelan® VE/K | N-Cocoylweizenproteinkondensat (INCI-Bezeichnung: Sodium Cocoyl Hydrolyzed Wheat Protein) (Zschimmer & Schwarz) |
| Texapon® NSO UP | Laurylethersulfat, Natriumsalz (ca. 27,5% Aktivsubstanz; INCI-Bezeichnung: Sodium Laureth Sulfate) (Cognis) |

### Mittel (B)

| | B1 | B2 | B3 |
|---|---|---|---|
| Zitronensäure (wasserfrei) | 0,5 | 0,5 | 0,5 |
| Natriumlaurylethersulfat (25 %ige wässrige Lsg) | 50,0 | 50,0 | 50,0 |
| Dinatriumdicocoampodicaetat | 7,0 | 7,0 | 7,0 |
| Salicylsäure | 0,2 | 0,2 | 0,2 |
| Natriumbenzoat | 0,5 | 0,5 | 0,5 |
| Euperlan PK 300 AM (ca. 60-64% Festkörper; INCI: Glycol Distearate, Glycerin, Laureth-4, Cocamidopropyl Betaine (Cognis)) | 2,0 | 2,0 | 2,0 |
| Cetiol HE (Kokosmonglycerid mit ca. 7,3 EO-Einheiten (INCI: PEG-7 Glyceryl Cocoate) (Cognis)) | 1,0 | 1,0 | 1,0 |
| **Oligopeptid**^{#} | **0,01** | **0,1** | **1,0** |
| Polyquaternium-10 | 0,5 | 0,5 | 0,5 |
| PEG-40 Hydrogenated Castor Oil | 1,0 | 1,0 | 1,0 |
| Natriumchlorid | 0,5 | 0,5 | 0,5 |
| Wasser | ad 100 | ad 100 | ad 100 |

| | | | |
|---|---|---|---|
| # Oligopeptid-Mischung der Formel (I), mit R1 = Formel (II), M1 = Formel (III), x = 1 - 10 und y = 1 - 10, M2 = Wasserstoff, mittleres Molgewicht 400 - 800 Dalton | | | |

| | B4 | B5 | B6 |
|---|---|---|---|
| Zitronensäure (wasserfrei) | 0,5 | 0,5 | 0,5 |
| Natriumlaurylethersulfat (25 %ige wässrige Lsg) | 50,0 | 50,0 | 50,0 |
| Dinatriumdicocoampodicaetat | 7,0 | 7,0 | 7,0 |
| Salicylsäure | 0,2 | 0,2 | 0,2 |
| Natriumbenzoat | 0,5 | 0,5 | 0,5 |
| Euperlan PK 300 AM (ca. 60-64% Festkörper; INCI: Glycol Distearate, Glycerin, Laureth-4, Cocamidopropyl Betaine (Cognis)) | 2,0 | 2,0 | 2,0 |
| Cetiol HE (Kokosmonglycerid mit ca. 7,3 EO-Einheiten (INCI: PEG-7 Glyceryl Cocoate) (Cognis)) | 1,0 | 1,0 | 1,0 |
| **2-Amino-3-(sulfosulfanyl)propansäure** | **0,01** | **0,1** | **1,0** |
| Polyquaternium-10 | 0,5 | 0,5 | 0,5 |
| PEG-40 Hydrogenated Castor Oil | 1,0 | 1,0 | 1,0 |
| Natriumchlorid | 0,5 | 0,5 | 0,5 |
| Wasser | ad 100 | ad 100 | ad 100 |

| | B7 | B7 | B9 |
|---|---|---|---|
| Paraffinum Liquidum | 1,0 | 1,0 | 1,0 |
| Dehyquart F75 (INCI-Bezeichnung: Distearoylethyl Hydroxyethylmonium Methosulfate, Cetearyl Alcohol) | 2,0 | 2,0 | 2,0 |
| **Oligopeptid**^{#} | **0,01** | **0,1** | **1,0** |
| Quaternium-87 | 1,5 | 1,5 | 1,5 |
| Cetearylalkohol | 3,5 | 3,5 | 3,5 |
| Propylparaben | 0,15 | 0,15 | 0,15 |
| Cetylpalmitat | 0,7 | 0,7 | 0,7 |
| Stearamidopropyldimethylamin | 1,0 | 1,0 | 1,0 |
| Trimethylhexadecylammoniumchlorid | 0,6 | 0,6 | 0,6 |
| Zitronensäure | 0,5 | 0,5 | 0,5 |
| Methylparaben | 0,15 | 0,15 | 0,15 |
| Wasser | ad 100 | ad 100 | ad 100 |

| | | | |
|---|---|---|---|
| # Oligopeptid-Mischung der Formel (I), mit R1 = Formel (II), M1 = Formel (III), x = 1 - 10 und y = 1 - 10, mittleres Molgewicht 400 - 800 Dalton | | | |

| | B10 | B11 | B12 |
|---|---|---|---|
| Paraffinum Liquidum | 1,0 | 1,0 | 1,0 |
| Dehyquart F75 (INCI-Bezeichnung: Distearoylethyl Hydroxyethylmonium Methosulfate, Cetearyl Alcohol) | 2,0 | 2,0 | 2,0 |
| **2-Amino-3-(sulfosulfanyl)propansäure** | **0,01** | **0,1** | **1,0** |
| Quaternium-87 | 1,5 | 1,5 | 1,5 |
| Cetearylalkohol | 3,5 | 3,5 | 3,5 |
| Propylparaben | 0,15 | 0,15 | 0,15 |
| Cetylpalmitat | 0,7 | 0,7 | 0,7 |
| Stearamidopropyldimethylamin | 1,0 | 1,0 | 1,0 |
| Trimethylhexadecylammoniumchlorid | 0,6 | 0,6 | 0,6 |
| Zitronensäure | 0,5 | 0,5 | 0,5 |
| Methylparaben | 0,15 | 0,15 | 0,15 |
| Wasser | ad 100 | ad 100 | ad 100 |

### Mittel (C)

| | C1 | C2 | C3 |
|---|---|---|---|
| Dipicolinsäure | 0,10 | 0,10 | 0,10 |
| Turpinal SL | 1,70 | 1,70 | 1,70 |
| Texapon NSO UP | 6,00 | 6,00 | 6,00 |
| Wasserstoffperoxid | 4,00 | 5,00 | 7,5 |
| Ammoniak (25%ige wässrige Lösung) | 0,70 | 0,70 | 0,70 |
| Wasser | ad 100 | ad 100 | ad 100 |

Verfahren, mit Abfolge der Schritte in der angegebenen Reihenfolge

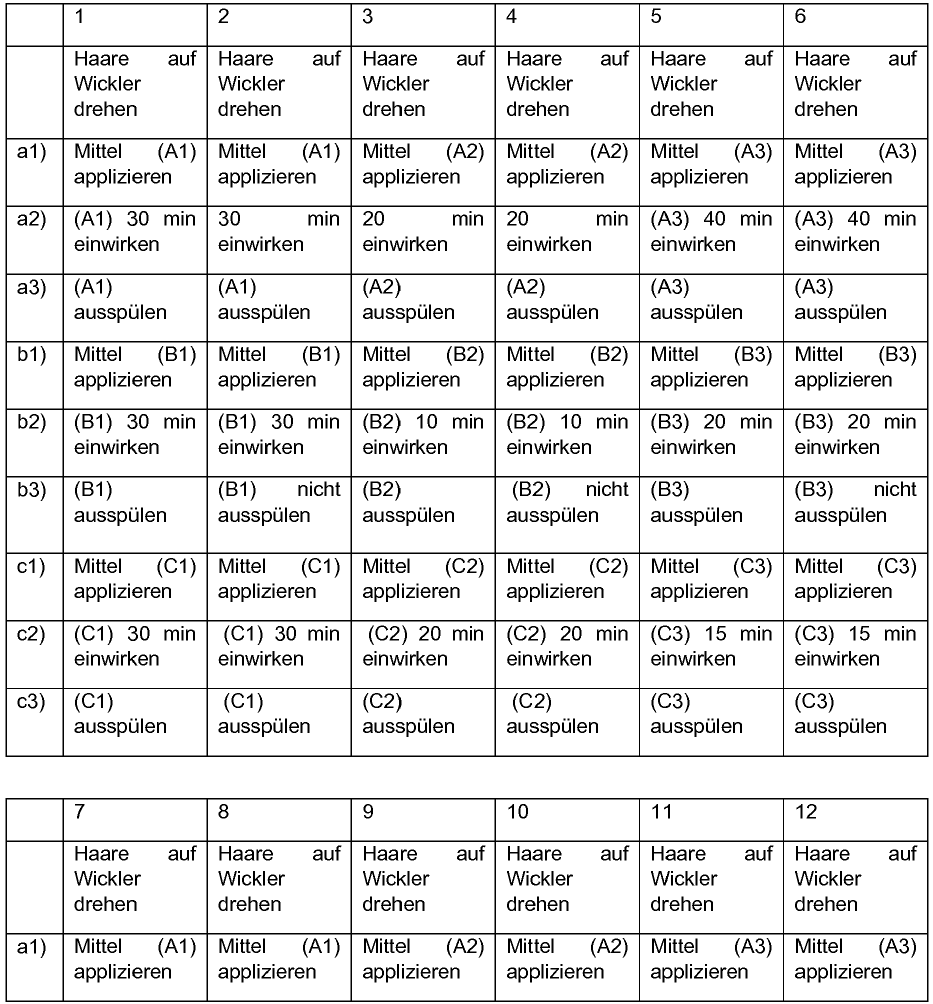

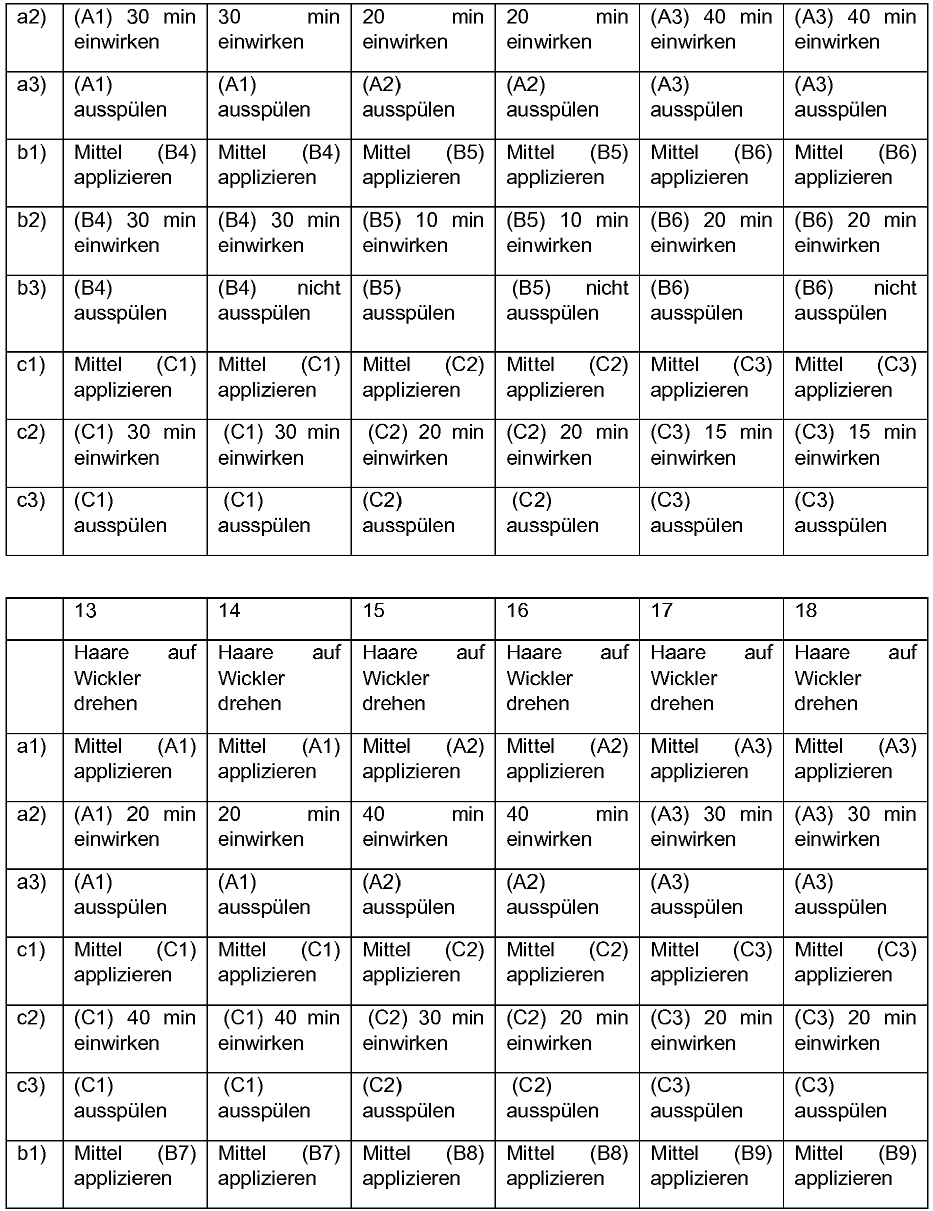

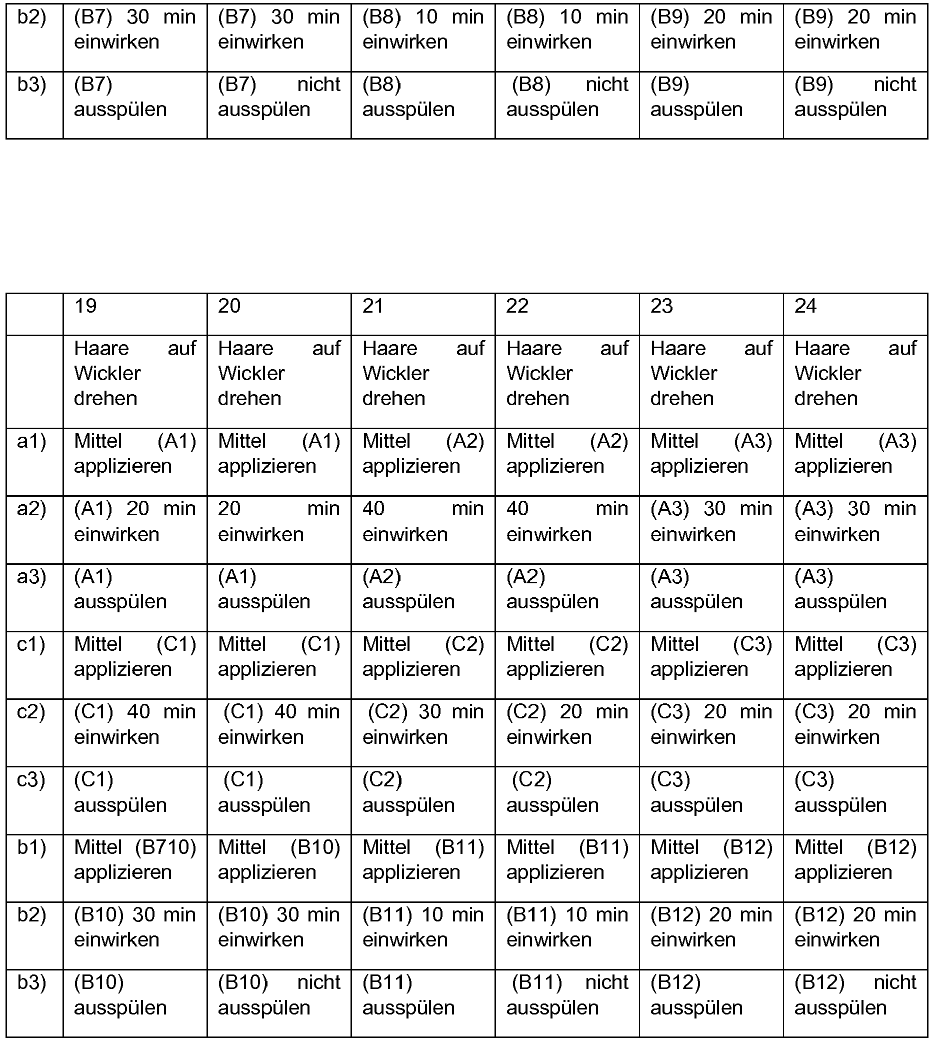

## Patentansprüche

1. Mehrkomponenten-Verpackungseinheit (Kit-of-Parts) zur permanenten Formveränderung von keratinischen Fasern, insbesondere menschlichen Haaren, umfassend getrennt voneinander konfektioniert
- einen Container (i) enthaltend ein kosmetisches Mittel (A) und
- einen Container (ii) enthaltend ein kosmetisches Mittel (B), und
- einen Container (iii) enthaltend ein kosmetisches Mittel (C), wobei
- das Mittel (A) in Container (i) Wasser enthält, einen pH-Wert von 7,5 bis 9,5 besitzt und mindestens eine Keratin reduzierende Verbindungen enthält, und
- das Mittel (B) in Container (ii) Wasser enthält, einen pH-Wert von 3,0 bis 5,5 besitzt und mindestens eine Verbindung der allgemeinen Formel (I) enthält 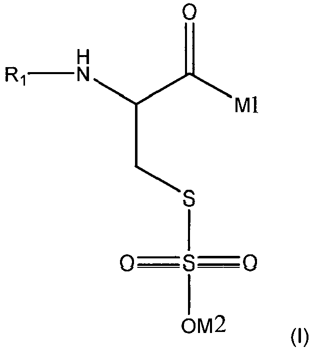 wobei
R1 steht für ein Wasserstoffatom oder für ein Strukturelement der Formel (II) 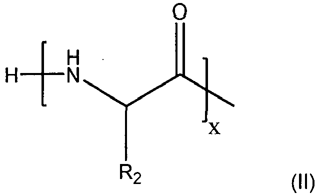 wobei
x für eine ganze Zahl von 1 bis 100 steht,
der Rest R2 in jedem der Strukturelemente der Formel (II) jeweils unabhängig vom vorangegangenen Strukturelement der Formel (II) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (III) 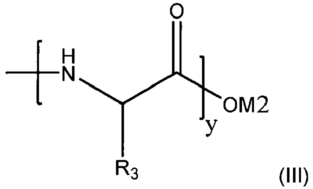
wobei
y für eine ganze Zahl von 1 bis 100 steht
der Rest R3 in jedem der Strukturelemente der Formel (III) jeweils unabhängig vom vorangegangenen Strukturelement der Formel (III) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺, und
- das Mittel (C) in Container (iii) Wasser enthält, einen pH-Wert von 2,5 bis 4,5 besitzt und mindestens ein Oxidationsmittel enthält.

2. Mehrkomponenten-Verpackungseinheit nach Anspruch 1, **dadurch gekennzeichnet, dass** das Mittel (A) in Container (i) mindestens eine Keratin reduzierende Verbindung aus der Gruppe aus Thioglycolsäure, Thiomilchsäure, Thioapfelsäure, Cystein, Cysteamin, Cystin, 2-Mercaptoethansulfonsäure und/oder deren physiologisch verträglichen Salzen enthält.

3. Mehrkomponenten-Verpackungseinheit nach Anspruch 1 bis 2, **dadurch gekennzeichnet, dass** das Mittel (A) in Container (i) eine oder mehrere Keratin reduzierende Verbindungen aus der Gruppe aus Thioglycolsäure, Ammoniumthioglycolat, Thiomilchsäure und/oder Ammoniumthiolactat enthält.

4. Mehrkomponenten-Verpackungseinheit nach Anspruch 1 bis 3, **dadurch gekennzeichnet, dass** dass das Mittel (A) in Container (i) - bezogen auf das Gesamtgewicht des Mittels (A) - ein oder mehrere Keratin reduzierende Verbindungen in einer Gesamtmenge von 1,5 bis 20,0 Gew.-%, bevorzugt 3,0 bis 15,0 Gew.-%, weiter bevorzugt von 4,0 bis 12,0 Gew.-% und besonders bevorzugt von 4,5 bis 12,5 Gew.-% enthält.

5. Mehrkomponenten-Verpackungseinheit nach Anspruch 1 bis 4, **dadurch gekennzeichnet, dass** das Mittel (B) in Container (ii) mindestens eine Verbindung der Formel (I) enthält, wobei
R1 für ein Wasserstoffatom steht und
M1 für eine Gruppierung -OM2 steht.

6. Mehrkomponenten-Verpackungseinheit nach Anspruch 1 bis 5, **dadurch gekennzeichnet, dass** das Mittel (B) in Container (ii) mindestens eine Verbindung der Formel (I) enthält, die ein Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

7. Mehrkomponenten-Verpackungseinheit nach Anspruch 1 bis 6, **dadurch gekennzeichnet, dass** das Mittel (B) in Container (ii) mindestens eine Verbindung der Formel (I) enthält, wobei
R1 für ein Strukturelement der Formel (II) steht, und
M1 für ein Strukturelement der Formel (III) steht, und
x für eine ganze Zahl von 1 bis 10 steht und
y für eine ganze Zahl von 1 bis 10 steht.

8. Mehrkomponenten-Verpackungseinheit nach Anspruch 1 bis 7, **dadurch gekennzeichnet, dass** das Mittel (B) in Container (ii) mindestens eine Verbindung der Formel (I) enthält, wobei
R1 für ein Strukturelement der Formel (II) steht, und
der Rest R2 in mindestens einem Strukturelement der Formel (II) für eine (Sulfosulfanyl)methylGruppe (d.h. eine Gruppe HO-S(O₂)-S-CH₂-)steht.

9. Mehrkomponenten-Verpackungseinheit nach Anspruch 1 bis 8, **dadurch gekennzeichnet, dass** das Mittel (B) in Container (ii) mindestens eine Verbindung der Formel (I) enthält, wobei
R1 für ein Strukturelement der Formel (II) steht, und
x für eine ganze Zahl von mindestens 3 steht und
der Rest R2 in mindestens einem Strukturelement der Formel (II) für eine 2-Carboxyethyl-Gruppe steht.

10. Mehrkomponenten-Verpackungseinheit nach Anspruch 1 bis 9, **dadurch gekennzeichnet, dass** das Mittel (B) in Container (ii) mindestens eine Verbindung der Formel (I) enthält, wobei
M1 für ein Strukturelement der Formel (III) steht, und
der Rest R3 in mindestens einem Strukturelement der Formel (III) für eine (Sulfosulfanyl)methylGruppe (d.h. eine Gruppe HO-S(O₂)-S-CH₂-)steht.

11. Mehrkomponenten-Verpackungseinheit nach Anspruch 1 bis 10, **dadurch gekennzeichnet, dass** das Mittel (B) in Container (ii) mindestens eine Verbindung der Formel (I) enthält, wobei
M1 für ein Strukturelement der Formel (III) steht, und
y für eine ganze Zahl von mindestens 3 steht und
der Rest R3 in mindestens einem Strukturelement der Formel (III) für eine 2-Carboxyethyl-Gruppe steht.

12. Mehrkomponenten-Verpackungseinheit nach Anspruch 1 bis 11, **dadurch gekennzeichnet, dass** das Mittel (B) in Container (ii) - bezogen auf das Gesamtgewicht des Mittels (B) - eine oder mehrere Verbindungen der Formel (I) in einer Gesamtenge von 0,0001 bis 10,0 Gew.-%, bevorzugt 0,001 bis 10,0 Gew.-%, weiter bevorzugt von 0,01 bis 2,5 Gew.-%, noch weiter bevorzugt von 0,05 bis 1,0 Gew.-% und besonders bevorzugt von 0,05 bis 0,3 Gew.-% enthält.

13. Mehrkomponenten-Verpackungseinheit nach Anspruch 1, **dadurch gekennzeichnet, dass** das Mittel (C) in Container (iii) als Oxidationsmittel Wasserstoffperoxid enthält.

14. Verfahren zur permanenten Formveränderung von keratinischen Fasern, insbesondere menschlichen Haaren, umfassend die folgenden Schritte
a1) Applizieren eines kosmetischen Mittels (A) auf die keratinischen Fasern,
a2) Einwirkenlassen des Mittels (A) für einen Zeitraum von 2 bis 60 Minuten,
a3) gegebenenfalls Ausspülen des Mittels (A) aus den keratinischen Fasern,
b1) Applizieren eines kosmetischen Mittels (B) auf die keratinischen Fasern,
b2) Einwirkenlassen des Mittels (B) für einen Zeitraum von 2 bis 60 Minuten,
b3) gegebenenfalls Ausspülen des kosmetischen Mittels (B) aus den keratinischen Fasern, c1) Applizieren eines kosmetischen Mittels (C) auf die keratinischen Fasern,
c2) Einwirkenlassen des Mittels (C) für einen Zeitraum von 2 bis 60 Minuten,
c3) Ausspülen des Mittels (C) aus den keratinischen Fasern,
wobei
- das Mittel (A) ein Mittel ist, wie es in den Ansprüchen 1 bis 4 beschrieben wurde
- das Mittel (B) ein Mittel ist, wie es in den Ansprüchen 1 und 5 bis 12 beschrieben wurde und
- das Mittel (C) ein Mittel ist, wie es in den Ansprüchen 1 und 13 beschrieben wurde.

15. Verfahren nach Anspruch 14, umfassend die folgenden Schritte in der angegebenen Reihenfolge:
a1) Applizieren eines kosmetischen Mittels (A) auf die keratinischen Fasern,
a2) Einwirkenlassen des Mittels (A) für einen Zeitraum von 2 bis 60 Minuten,
a3) Ausspülen des Mittels (A) aus den keratinischen Fasern,
b1) Applizieren eines kosmetischen Mittels (B) auf die keratinischen Fasern,
b2) Einwirkenlassen des Mittels (B) für einen Zeitraum von 2 bis 60 Minuten,
b3) gegebenenfalls Ausspülen des kosmetischen Mittels (B) aus den keratinischen Fasern,
c1) Applizieren eines kosmetischen Mittels (C) auf die keratinischen Fasern,
c2) Einwirkenlassen des Mittels (C) für einen Zeitraum von 2 bis 60 Minuten,
c3) Ausspülen des Mittels (C) aus den keratinischen Fasern,

16. Verfahren nach Anspruch 14, umfassend die folgenden Schritte in der angegebenen Reihenfolge:
a1) Applizieren eines kosmetischen Mittels (A) auf die keratinischen Fasern,
a2) Einwirkenlassen des Mittels (A) für einen Zeitraum von 2 bis 60 Minuten,
a3) Ausspülen des Mittels (A) aus den keratinischen Fasern,
c1) Applizieren eines kosmetischen Mittels (C) auf die keratinischen Fasern,
c2) Einwirkenlassen des Mittels (C) für einen Zeitraum von 2 bis 60 Minuten,
c3) Ausspülen des Mittels (C) aus den keratinischen Fasern,
b1) Applizieren eines kosmetischen Mittels (B) auf die keratinischen Fasern,
b2) Einwirkenlassen des Mittels (B) für einen Zeitraum von 2 bis 60 Minuten,
b3) gegebenenfalls Ausspülen des kosmetischen Mittels (B) aus den keratinischen Fasern.

17. Verfahren nach einem der Ansprüche 14 bis 16, **dadurch gekennzeichnet, dass** die keratinischen Fasern unter Zuhilfenahme von Verformungshilfsmitteln vor, nach oder während dem Schritt a1), a2) oder a3) verformt werden.

## Claims

1. A multi-component packaging unit (kit-of-parts) for permanently changing the shape of keratin fibers, in particular human hair, comprising, packaged separately from one another,
- a container (i) containing a cosmetic agent (A), and
- a container (ii) containing a cosmetic agent (B), and
- a container (iii) containing a cosmetic agent (C), wherein
- the agent (A) in container (i) contains water, has a pH of from 7.5 to 9.5 and contains at least one keratin-reducing compound, and
- the agent (B) in container (ii) contains water, has a pH of from 3.0 to 5.5 and contains at least one compound of general formula (I) 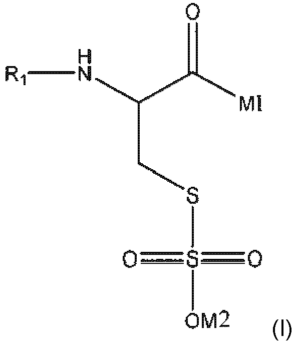 where
R1 represents a hydrogen atom or a structural element of formula (II) 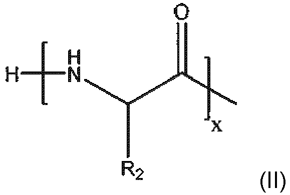 where
x represents an integer of from 1 to 100,
the functional group R2 in each of the structural elements of formula (II) can be selected independently of the preceding structural element of formula (II) in each case,
R2 represents a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1 H-imidazol-4-ylmethyl group, a 1 H-indol-3-ylmethyl group or a (sulfosulfanyl)methyl group,
M1 represents the grouping -OM2 or a structural element of formula (III) 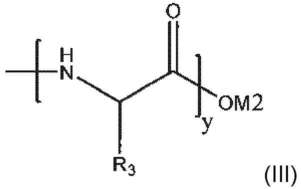
where
y represents an integer of from 1 to 100,
the functional group R3 in each of the structural elements of formula (III) can be selected independently of the preceding structural element of formula (III) in each case,
R3 represents a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1H-imidazol-4-ylmethyl group, a 1 H-indol-3-ylmethyl group or a (sulfosulfanyl)methyl group,
M2 represents a hydrogen atom, an equivalent of a monovalent or polyvalent cation or an ammonium ion (NH₄)⁺, and
- the agent (C) in container (iii) contains water, has a pH of from 2.5 to 4.5 and contains at least one oxidizing agent.

2. The multi-component packaging unit according to claim 1, **characterized in that** the agent (A) in container (i) contains at least one keratin-reducing compound from the group consisting of thioglycolic acid, thiolactic acid, thiomalic acid, cysteine, cysteamine, cystine, 2-mercaptoethanesulfonic acid and/or the physiologically acceptable salts thereof.

3. The multi-component packaging unit according to claims 1 to 2, **characterized in that** the agent (A) in container (i) contains one or more keratin-reducing compounds from the group consisting of thioglycolic acid, ammonium thioglycolate, thiolactic acid and/or ammonium thiolactate.

4. The multi-component packaging unit according to claims 1 to 3, **characterized in that** the agent (A) in container (i) contains one or more keratin-reducing compounds in a total amount of from 1.5 to 20.0 wt.%, preferably from 3.0 to 15.0 wt.%, more preferably from 4.0 to 12.0 wt.% and particularly preferably from 4.5 to 12.5 wt.%, based on the total weight of the agent (A).

5. The multi-component packaging unit according to claims 1 to 4, **characterized in that** the agent (B) in container (ii) contains at least one compound of formula (I), where
R1 represents a hydrogen atom, and
M1 represents a grouping -OM2.

6. The multi-component packaging unit according to claims 1 to 5, **characterized in that** the agent (B) in container (ii) contains at least one compound of formula (I) which has a molecular weight M_{w} of from 200 to 2,000 Da, preferably from 250 to 1,500 Da, more preferably from 300 to 1,200 Da, in particular from 400 to 800 Da.

7. The multi-component packaging unit according to claims 1 to 6, **characterized in that** the agent (B) in container (ii) contains at least one compound of formula (I), where
R1 represents a structural element of formula (II), and
M1 represents a structural element of formula (III), and
x represents an integer of from 1 to 10, and
y represents an integer of from 1 to 10.

8. The multi-component packaging unit according to claims 1 to 7, **characterized in that** the agent (B) in container (ii) contains at least one compound of formula (I), where
R1 represents a structural element of formula (II), and
the functional group R2 in at least one structural element of formula (II) represents a (sulfosulfanyl)methyl group (i.e. a group HO-S(O₂)-S-CH₂-).

9. The multi-component packaging unit according to claims 1 to 8, **characterized in that** the agent (B) in container (ii) contains at least one compound of formula (I), where
R1 represents a structural element of formula (II), and
x represents an integer of at least 3, and
the functional group R2 in at least one structural element of formula (II) represents a 2-carboxyethyl group.

10. The multi-component packaging unit according to claims 1 to 9, **characterized in that** the agent (B) in container (ii) contains at least one compound of formula (I), where
M1 represents a structural element of formula (III), and
the functional group R3 in at least one structural element of formula (III) represents a (sulfosulfanyl)methyl group (i.e. a group HO-S(O₂)-S-CH₂-).

11. The multi-component packaging unit according to claims 1 to 10, **characterized in that** the agent (B) in container (ii) contains at least one compound of formula (I), where
M1 represents a structural element of formula (III), and
y represents an integer of at least 3, and
the functional group R3 in at least one structural element of formula (III) represents a 2-carboxyethyl group.

12. The multi-component packaging unit according to claims 1 to 11, **characterized in that** the agent (B) in container (ii) contains one or more compounds of formula (I) in a total amount of from 0.0001 to 10.0 wt.%, preferably from 0.001 to 10.0 wt.%, more preferably from 0.01 to 2.5 wt.%, even more preferably from 0.05 to 1.0 wt.%, and particularly preferably from 0.05 to 0.3 wt.%, based on the total weight of the agent (B).

13. The multi-component packaging unit according to claim 1, **characterized in that** the agent (C) in container (iii) contains hydrogen peroxide as the oxidizing agent.

14. A method for permanently changing the shape of keratin fibers, in particular human hair, comprising the following steps:
a1) applying a cosmetic agent (A) to the keratin fibers,
a2) allowing the agent (A) to act for a period of from 2 to 60 minutes,
a3) optionally rinsing out the agent (A) from the keratin fibers,
b1) applying a cosmetic agent (B) to the keratin fibers,
b2) allowing the agent (B) to act for a period of from 2 to 60 minutes,
b3) optionally rinsing out the cosmetic agent (B) from the keratin fibers,
c1) applying a cosmetic agent (C) to the keratin fibers,
c2) allowing the agent (C) to act for a period of from 2 to 60 minutes,
c3) rinsing out the agent (C) from the keratin fibers,
wherein
- the agent (A) is an agent as described in claims 1 to 4,
- the agent (B) is an agent as described in claims 1 and 5 to 12, and
- the agent (C) is an agent as described in claims 1 and 13.

15. The method according to claim 14, comprising the following steps in the stated sequence:
a1) applying a cosmetic agent (A) to the keratin fibers,
a2) allowing the agent (A) to act for a period of from 2 to 60 minutes,
a3) rinsing out the agent (A) from the keratin fibers,
b1) applying a cosmetic agent (B) to the keratin fibers,
b2) allowing the agent (B) to act for a period of from 2 to 60 minutes,
b3) optionally rinsing out the cosmetic agent (B) from the keratin fibers,
c1) applying a cosmetic agent (C) to the keratin fibers,
c2) allowing the agent (C) to act for a period of from 2 to 60 minutes,
c3) rinsing out the agent (C) from the keratin fibers,

16. The method according to claim 14, comprising the following steps in the stated sequence:
a1) applying a cosmetic agent (A) to the keratin fibers,
a2) allowing the agent (A) to act for a period of from 2 to 60 minutes,
a3) rinsing out the agent (A) from the keratin fibers,
c1) applying a cosmetic agent (C) to the keratin fibers,
c2) allowing the agent (C) to act for a period of from 2 to 60 minutes,
c3) rinsing out the agent (C) from the keratin fibers,
b1) applying a cosmetic agent (B) to the keratin fibers,
b2) allowing the agent (B) to act for a period of from 2 to 60 minutes,
b3) optionally rinsing out the cosmetic agent (B) from the keratin fibers.

17. The method according to one of claims 14 to 16, **characterized in that** the keratin fibers are shaped using shaping aids before, after or during step a1), a2) or a3).

## Revendications

1. Unité de conditionnement multi-composant (kit) pour la mise en forme permanente de fibres kératiniques, en particulier des cheveux humains, comprenant, conditionnés séparément les uns des autres
- un récipient (i) contenant un agent cosmétique (A) et
- un récipient (ii) contenant un agent cosmétique (B), et
- un récipient (iii) contenant un agent cosmétique (C),
- l'agent (A) dans le récipient (i) contenant de l'eau, ayant un pH de 7,5 à 9,5 et contenant au moins un composé réducteur de kératine, et
- l'agent (B) dans le récipient (ii) contenant de l'eau, ayant un pH de 3,0 à 5,5 et contenant au moins un composé de formule générale (I) 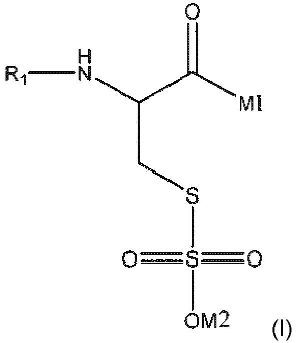 où
R1 représente un atome d'hydrogène ou un élément structurel de formule (II) 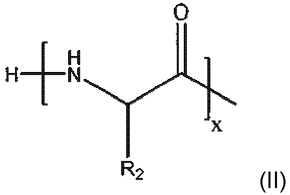 où
x représente un nombre entier de 1 à 100,
le radical R2 dans chacun des éléments structurels de formule (II) peut être choisi dans chaque cas indépendamment de l'élément structurel précédent de formule (II), R2 représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl) méthyle,
M1 représente le groupement -OM2 ou un élément structurel de formule (III) 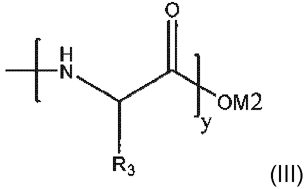
où
y représente un nombre entier de 1 à 100,
le radical R3 dans chacun des éléments structurels de formule (III) peut être choisi dans chaque cas indépendamment de l'élément structurel précédent de formule (III),
R3 représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl) méthyle,
M2 représente un atome d'hydrogène, un équivalent d'un cation monovalent ou polyvalent ou un ion ammonium (NH₄)⁺, et
- l'agent (C) dans le récipient (iii) contenant de l'eau, ayant un pH de 2,5 à 4,5 et contenant au moins un agent oxydant.

2. Unité de conditionnement multi-composant selon la revendication 1, **caractérisée en ce que** l'agent (A) dans le récipient (i) contient au moins un composé réducteur de kératine issu du groupe constitué de l'acide thioglycolique, de l'acide thiolactique, de l'acide thiomalique, de la cystéine, de la cystéamine, de la cystine, de l'acide 2-mercaptoéthanesulfonique et/ou de leurs sels physiologiquement acceptables.

3. Unité de conditionnement multi-composant selon la revendication 1 à 2, **caractérisée en ce que** l'agent (A) dans le récipient (i) contient un ou plusieurs composés réducteurs de kératine issu du groupe constitué de l'acide thioglycolique, du thioglycolate d'ammonium, de l'acide thiolactique et/ou du thiolactate d'ammonium.

4. Unité de conditionnement multi-composant selon la revendication 1 à 3, **caractérisée en ce que** l'agent (A) dans le récipient (i) contient - par rapport au poids total de l'agent (A) - un ou plusieurs composés réducteurs de kératine en une quantité totale de 1,5 à 20,0 % en poids, de préférence de 3,0 à 15,0 % en poids, de manière davantage préférée de 4,0 à 12,0 % en poids et de manière particulièrement préférée de 4,5 à 12,5 % en poids.

5. Unité de conditionnement multi-composant selon la revendication 1 à 4, **caractérisée en ce que** l'agent (B) dans le récipient (ii) contient au moins un composé de formule (I), où
R1 représente un atome d'hydrogène et
M1 représente un groupement -OM2.

6. Unité de conditionnement multi-composant selon la revendication 1 à 5, **caractérisée en ce que** l'agent (B) dans le récipient (ii) contient au moins un composé de formule (I) qui a un poids moléculaire Mw de 200 à 2 000 Da, de préférence de 250 à 1 500 Da, de manière préférée de 300 à 1 200 Da, en particulier de 400 à 800 Da.

7. Unité de conditionnement multi-composant selon la revendication 1 à 6, **caractérisée en ce que** l'agent (B) dans le récipient (ii) contient au moins un composé de formule (I), où
R1 représente un élément structurel de formule (II), et
M1 représente un élément structurel de formule (III), et
x représente un nombre entier de 1 à 10, et
y représente un nombre entier de 1 à 10.

8. Unité de conditionnement multi-composant selon la revendication 1 à 7, **caractérisée en ce que** l'agent (B) dans le récipient (ii) contient au moins un composé de formule (I), où
R1 représente un élément structurel de formule (II), et
le radical R2 dans au moins un élément structurel de formule (II) représente un groupe (sulfosulfanyl)méthyle (c'est-à-dire un groupe HO-S(O₂)-S-CH₂-).

9. Unité de conditionnement multi-composant selon la revendication 1 à 8, **caractérisée en ce que** l'agent (B) dans le récipient (ii) contient au moins un composé de formule (I), où
R1 représente un élément structurel de formule (II), et
x représente un nombre entier au moins égal à 3 et
le radical R2 représente, dans au moins un élément structurel de formule (II), un groupe 2-carboxyéthyle.

10. Unité de conditionnement multi-composant selon la revendication 1 à 9, **caractérisée en ce que** l'agent (B) dans le récipient (ii) contient au moins un composé de formule (I), où
M1 représente un élément structurel de formule (III), et
le radical R3 représente, dans au moins un élément structurel de formule (III), un groupe (sulfosulfanyl)méthyle (c'est-à-dire un groupe HO-S(O₂)-S-CH₂-).

11. Unité de conditionnement multi-composant selon la revendication 1 à 10, **caractérisée en ce que** l'agent (B) dans le récipient (ii) contient au moins un composé de formule (I), où
M1 représente un élément structurel de formule (III), et
y représente un nombre entier au moins égal à 3 et
le radical R3 représente, dans au moins un élément structurel de formule (III), un groupe 2-carboxyéthyle.

12. Unité de conditionnement multi-composant selon la revendication 1 à 11, **caractérisée en ce que** l'agent (B) dans le récipient (ii) contient - par rapport au poids total de l'agent (B) - un ou plusieurs composés de formule (I) en une quantité totale de 0,0001 à 10,0 % en poids, de préférence de 0,001 à 10,0 % en poids, de manière davantage préférée de 0,01 à 2,5 % en poids, de manière encore davantage préférée de 0,05 à 1,0 % en poids et de manière particulièrement préférée de 0,05 à 0,3 % en poids.

13. Unité de conditionnement multi-composant selon la revendication 1, **caractérisée en ce que** l'agent (C) dans le récipient (iii) contient du peroxyde d'hydrogène comme agent oxydant.

14. Procédé de mise en forme permanente de fibres kératiniques, en particulier de cheveux humains, comprenant les étapes suivantes :
a1) appliquer un agent cosmétique (A) sur les fibres kératiniques,
a2) laisser agir l'agent (A) pendant une période de 2 à 60 minutes,
a3) éliminer éventuellement l'agent (A) des fibres kératiniques en le rinçant,
b1) appliquer un agent cosmétique (B) sur les fibres kératiniques,
b2) laisser agir l'agent (B) pendant une période de 2 à 60 minutes,
b3) éliminer éventuellement l'agent cosmétique (B) des fibres kératiniques en le rinçant,
c1) appliquer un agent cosmétique (C) sur les fibres kératiniques,
c2) laisser agir l'agent (C) pendant une période de 2 à 60 minutes,
c3) éliminer l'agent (C) des fibres kératiniques en le rinçant,
dans lequel
- l'agent (A) est un agent tel que décrit dans les revendications 1 à 4
- l'agent (B) est un agent tel que décrit dans les revendications 1 et 5 à 12, et
- l'agent (C) est un agent tel que décrit dans les revendications 1 et 13.

15. Procédé selon la revendication 14, comprenant les étapes suivantes dans l'ordre indiqué :
a1) appliquer un agent cosmétique (A) sur les fibres kératiniques,
a2) laisser agir l'agent (A) pendant une période de 2 à 60 minutes,
a3) éliminer l'agent (A) des fibres kératiniques en le rinçant,
b1) appliquer un agent cosmétique (B) sur les fibres kératiniques,
b2) laisser agir l'agent (B) pendant une période de 2 à 60 minutes,
b3) éliminer éventuellement l'agent cosmétique (B) des fibres kératiniques en le rinçant,
c1) appliquer un agent cosmétique (C) sur les fibres kératiniques,
c2) laisser agir l'agent (C) pendant une période de 2 à 60 minutes,
c3) éliminer l'agent (C) des fibres kératiniques en le rinçant.

16. Procédé selon la revendication 14, comprenant les étapes suivantes dans l'ordre indiqué :
a1) appliquer un agent cosmétique (A) sur les fibres kératiniques,
a2) laisser agir l'agent (A) pendant une période de 2 à 60 minutes,
a3) éliminer l'agent (A) des fibres kératiniques en le rinçant,
c1) appliquer un agent cosmétique (C) sur les fibres kératiniques,
c2) laisser agir l'agent (C) pendant une période de 2 à 60 minutes,
c3) éliminer l'agent (C) des fibres kératiniques en le rinçant,
b1) appliquer un agent cosmétique (B) sur les fibres kératiniques,
b2) laisser agir l'agent (B) pendant une période de 2 à 60 minutes,
b3) éliminer éventuellement l'agent cosmétique (B) des fibres kératiniques en le rinçant.

17. Procédé selon l'une des revendications 14 à 16, **caractérisé en ce que** les fibres kératiniques sont mises en forme à l'aide de moyens d'aide à la mise en forme avant, après ou pendant l'étape a1), a2) ou a3).
